# FASCICULE DE BREVET EUROPEEN

(11) **EP 0 834 502 B1**
(45) Date de publication et mention de la délivrance du brevet: **28.12.2005**
(21) Numéro de dépôt: 97402311.1
(22) Date de dépôt: 02.10.1997
(51) Int. Cl.: C07C 311/48, C08F 14/16, C08F 28/02, C08G 77/04, G03F 7/029, G03F 7/039, C08F 2/46

(54) **Composés polymères polyioniques, leur procédé de préparation et leur utilisation comme photoamorceurs**
Polymerische polyionische Verbindungen, Verfahren zu deren Herstellung sowie deren Anwendung als Photoinitiatoren
Polymeric polyionic compounds, process for their preparation and their use as photoinitiators

(30) Priorité: 03.10.1996 CA 2187046
(43) Date de publication de la demande: 08.04.1998
(73) Titulaire: HYDRO-QUEBEC, Montréal Québec H2Z 1A4 (CA); CENTRE NATIONAL DE LA RECHERCHE SCIENTIFIQUE (CNRS), 75016 Paris (FR)
(72) Inventeur: Vallee, Alain, Varennes, Québec, J3X 1Y9 (CA); Armand, Michel, Montréal, Québec, H3T 1N2 (CA); Ollivrin, Xavier, 38000 Grenoble (FR); Michot, Christophe, 38000 Grenoble (FR)
(74) Mandataire: Sueur, Yvette

(56) Documents cités:
- EP-A- 0 703 236
- US-A- 4 197 174
- US-A- 4 780 511
- US-A- 5 340 898
- US-A- 5 550 171
- US-A- 5 554 664

## Description

La présente invention concerne des composés polyioniques, leur procédé de préparation et leur utilisation comme photoamorceurs pour la polymérisation ou la réticulation par voie cationique de monomères et de prépolymères, ou pour la modification des paramètres de solubilité de certains polymères qui peuvent être utilisés comme photorésists.

Une polymérisation mettant en jeu un mécanisme de type cationique présente de nombreux avantages. En particulier, elle est rapide, même à basse température, le taux d'utilisation du monomère est élevé et la sensibilité aux contaminants atmosphériques, notamment l'oxygène, est faible par comparaison aux polymérisations radicalaires ou anioniques.

Les monomères, les prépolymères et les polymères contenant des fonctions époxydes cycloaliphatiques et les éthers vinyliques sont utilisés de plus en plus notamment dans l'industrie des peintures, des vernis, des encres, des colles et des supports anti-adhésifs. De plus, les éthers vinyliques apparaissent d'une manière générale dénués de toxicité, contrairement aux acrylates ou aux méthacrylates. Les monomères et les prépolymères du type époxydes ou du type éther vinylique peuvent être polymérisés selon différentes méthodes, la polymérisation cationique étant particulièrement intéressante.

Les catalyseurs de polymérisation cationique sont d'une manière générale des acides au sens de Brønsted HX (donneurs de protons), ou des acides au sens de Lewis (accepteurs de doublets électroniques), ces derniers fonctionnant en présence d'un co-catalyseur source de protons. Il est nécessaire que ces acides soient suffisamment forts pour assurer la stabilité de l'espèce cationique portée soit par le monomère, soit par la chaîne macromoléculaire en croissance, ce qui signifie que l'anion correspondant X⁻ doit posséder le pouvoir nucléophile le plus faible possible. Les acides de BrØnsted les plus utilisés comme catalyseurs de polymérisation cationique sont CF₃SO₃H, HClO₄, HBF₄, HPF₆, HAsF₆ et HSbF₆. Ces acides se classent comme suit, en ce qui concerne les vitesses d'amorçage, de propagation ainsi que l'obtention des masses moléculaires les plus élevées :

CF₃SO₃H < HClO₄ ≈ HBF₄ < HPF₆ ≈ HAsF₆ ≈ HSbF₆.

Plus récemment, on a également utilisé des composés à caractère acide tels que le bis(perfluoroalkylsulfonyl)-imide (US-A-4,031,036, Koshar, et al) ou le bis(perfluoroalkylsulfonyl)méthane (US-A-3,632,843, Allen et al.).

Il est connu que la préparation in-situ de catalyseurs de polymérisation présente de nombreux avantages. La production in situ de l'acide capable de catalyser la réticulation d'un monomère permet en effet de mettre en forme un monomère ou un prépolymère fluide (matériau thermoplastique ou solution) et de lui donner ses propriétés définitives par exemple par simple action d'un rayonnement. Cette technique est très utilisée pour les encres, les peintures, les films adhésifs et les films anti-adhésifs. Il faut noter en outre que la préparation de l'acide in situ à partir d'un sel permet dans de nombreux cas d'éviter le stockage et la manipulation de composés acides plus corrosifs que les sels correspondants.

Les catalyseurs peuvent être préparés in situ par action de chaleur. Par exemple, des sels d'ammonium ou de métal de bis (perfluoroalkylsulfonyl) imide (US-A-4, 031,036, Koshar, et al.) ou des sels d'ammonium ou d'amine de bis-(perfluoroalkylsulfonyl) méthane (US-A-3, 632,843, Allen et al) ont été utilisés pour obtenir in-situ, par chauffage, le bis(perfluoroalkylsulfonyl)imide ou le bis(perfluoroalkylsulfonyl)méthane correspondant, agissant ensuite comme catalyseur. Ces catalyseurs, dits "latents", ne présentent cependant qu'un intérêt limité du fait de la nécessité d'un chauffage prolongé à température élevée pour obtenir la libération de l'acide, cette libération étant en outre progressive, et non pas intégrale à l'initiation. Il en résulte d'une part une faible vitesse de réaction, et d'autre part des polymères de qualité médiocre en ce qui concerne la masse moléculaire, la polydispersité et la coloration.

Les catalyseurs acides peuvent également être préparés in situ par action d'un rayonnement actinique (tel que les photons dont la longueur d'onde correspond au rayonnement ultraviolet, visible, γ et X), ou d'un rayonnement β (faisceau d'électrons) sur un sel approprié. Un tel sel, qui présente une labilité chimique sous l'action d'un rayonnement actinique ou d'un rayonnement β amenant la libération de l'acide correspondant ayant une forte activité catalytique, est un photoamorceur. Les avantages d'un tel procédé sont multiples : la libération du catalyseur par irradiation est rapide et pratiquement totale, ce qui entraîne une initiation simultanée de la croissance des chaînes, et donc un répartition des masses plus homogène avec une moindre polydispersité, et de meilleures propriétés mécaniques. La polymérisation peut être effectuée à une température relativement basse, ce qui évite une décomposition ou une coloration des matériaux obtenus, ainsi que la formation de bulles lorsqu'un solvant est utilisé ou lorsque le mélange réactionnel contient un additif volatil que l'on souhaite conserver dans le matériau final et qui joue le rôle de plastifiant.

On connaît la capacité de différents sels (hexafluoro-antimonates, hexafluoroarséniates, hexafluoroplatinates et tétrafluoroborates) d'aryldiazonium, d'aryliodonium, d'arylsulfonium, d'arylacylsulfonium ou d'arèneferrocénium à former, sous l'action de radiations actiniques, des acides (respectivement HSbF₆, HAsF₆, HPF₆, HBF₄) utilisables comme catalyseurs de polymérisation cationique. La préparation et l'utilisation de tels sels d'iodonium comme photoamorceurs dans des réactions de polymérisation cationique est décrite notamment dans US-4,780,511. La préparation de tels sels de sulfonium et leur utilisation comme photoamorceur sont décrites noteamment dans US-5,550,171. Néanmoins, tous ces sels présentent une toxicité non négligeable, associée principalement à l'élément central de la partie anionique, Sb, As, P et B ainsi qu'aux ions fluor pouvant être libérés au cours de la réaction de photolyse ou lors du traitement ultérieur du polymère (fusion, extrusion...). Pour fixer un ordre de grandeur, l'hexafluoroantimonate de diphényliodonium possède un LD50 de 40 mg/kg (mesuré selon le test n°10929 TAR) et entre dans la catégorie des produits classifiés "hautement toxiques".

D'autres sels contenant des cations de la même nature, mais des anions moins toxiques ont alors été proposés. Ainsi, US-A-5,554,664 décrit des sels dont l'anion est choisi parmi les tris(alkylsulfonyl)méthylures, les tris-(arylsulfonyl)méthylures, les bis (alkylsulfonyl) imidures et les bis(arylsulfonyl)imidures dans lesquels le groupe alkyle ou le groupe aryle est perfluoré ou fortement fluoré, et dont le cation est un iodonium, un sulfonium ou un organométallique. Ces composés peuvent être utilisés notamment comme initiateurs de polymérisation après activation in situ. Lorsque ces sels sont utilisés comme photoamorceurs de polymérisation, ils laissent, après leur décomposition initiée par le rayonnement actinique, des fragments qui peuvent diffuser à la surface du matériau et en modifier les propriétés chimiques, l'adhésion ou l'apparence d'une manière notable. Dans les cas des sels de sulfonium, ces résidus contiennent des thiols et des thioéthers dont l'odeur repoussante est perceptible à des taux extrêment faibles, ce qui limite l'utilisation de ces sels à des applications particulières. Ces composés sont des plus corrosifs vis à vis des métaux comme le cuivre ou divers composants de la microélectronique. Ainsi Kukzynski (USA 5,550,171) considère que la diffusion des résidus catalytiques est la cause principale de défaillance des disques de stockage informatique.

Des polymères photosensibles constitués par l'association d'un polycation polydiazonium et d'un polyanion polysulfonate sont décrits dans US-A-5,527,655. De tels polymères sont utilisés pour augmenter l'efficacité de réticulation. La solubilité de ces complexes n'est obtenue qu'avec des teneurs en diazonium nettement inférieures à 10% en poids en uniquement en présence d'un sel d'ammonium quaternaire agissant pour diminuer les interactions electrostatiques. US-A-5,534,623 décrit une composition à base de polydiazonium associé à des contre-ions du type PF₆ et destiné à la préparation de photorésists. Ces anions sont néanmoins toxiques et non compatibles avec la microélectronique car contenant un élement suceptible de contaminer le silicium (B, P, As ou Sb).

Il est également connu d'utiliser des acides générés à l'aide d'un rayonnement actinique pour dégrader les résines contenues dans un film constituant un photorésist. Cette technique est particulièrement efficace pour les photorésists à amplification chimique, dans lesquels de très faibles quantités de protons catalysent la décomposition de groupements tels que des esters contenant un groupement dérivé d'un alcool tertiaire (tel que par exemple un groupement tertiobutyle), faisant partie d'une chaîne macromoléculaire. Elle permet ainsi de modifier les paramètres de solubilité de la résine exposée à un rayonnement actinique et d'effectuer des opérations de masquage et de gravure sélectives telles qu'utilisées en microélectronique.

Lorsqu'une composition de photoresists ou une composition de résine à amplification chimique utilisée en microlithographie contient un photoamorceur, on considère que la diffusion des espèces ioniques de l'initiateur ou de l'acide formé détermine la limite de résolution spaciale, qui est de plusieurs dizaines de microns avec les initiateurs non polymères. Or il est demandé actuellement des résolutions inférieures à 1 micron pour l'industrie électroniques des microprocesseurs et des mémoires.

Les inventeurs ont maintenant trouvé de nouveaux composés ioniques qui permettent d'obtenir sous l'action d'un rayonnement actinique ou d'un rayonnement β, des acides qui se révèlent bons catalyseurs de polymérisation cationique ou de modification de polymères. Ces matériaux, contrairement à ce qui est attendu du comportement inhérent aux polyélectrolytes, c'est à dire une solubilité limitée à des solvants de très forte polarité, sont solubles ou dispersables dans les solvants organiques habituels ou les monomères destinés a être polymérisés ou leurs mélanges.

La présente invention a ainsi pour objet une nouvelle famille de composés, un procédé pour leur préparation, ainsi que leur utilisation comme photoamorceurs pour la polymérisation ou la réticulation de monomères par voie cationique, ou pour la modification de polymères, notamment lorsqu'ils sont utilisés comme photorésists.

Un composé de la présente invention est un composé ionique oligomère ou polymère constitué par une partie polycationique (A⁺)ₚ comprenant plusieurs unités ionium et par un nombre d'anions X⁻ suffisant pour assurer la neutralité électrique du composé, et il est caractérisé en ce que :
- les unités onium sont choisies dans le groupe constitué par les biaryliodonium, les arylsulfonium, les arylacylsulfonium, les diazonium, les cations organométalliques comprenant un métal de transition complexé par au moins un cycle insaturé comprenant de 4 à 12 atomes de carbone ;
- X⁻ est un anion imidure [R_{F}SO₂NSO₂R'_{F}]⁻ ou un anion méthylure [R_{F}SO₂C (Q) SO₂R'_{F}] ⁻ dans lesquels

1) Q représente :
   - H-, Cl-, F-, Br- ou CN- ;
   - un radical alkyle ayant de 1 à 30 atomes de carbone ;
   - un radical aryle ou alkylaryle ou arylalkyle ayant de 6 à 30 atomes de carbone ;
   - un groupe R"_{F} ou un groupe R"_{F}SO₂ ;
2) R_{F} et R'_{F}, ainsi que R"_{F} le cas échéant lorsque X⁻ est un anion méthylure, sont choisis indépendamment l'un de l'autre dans le groupe constitué par le fluor, les groupements perhaloalkyles ayant de 1 à 30 atomes de carbone, les groupements (perhaloalkyl)alkyloxy, les groupements cycloaliphatiques perhalogénés ayant de 3 à 30 atomes de carbone contenant éventuellement des hétéroatomes choisis parmi O et N, et/ou portant éventuellement au moins un chaînon perhaloalkyle, les groupements aryles perhalogénés ayant de 6 à 30 atomes de carbone ; ou bien
3) R_{F} et R'_{F} forment ensemble un radical divalent formant un cycle respectivement avec le groupe -SO₂-N-SO₂- ou avec le groupe -SO₂-C(Q)-SO₂-, ou bien, lorsque X⁻ est un anion méthylure, R"_{F} forme avec l'un des radicaux R_{F} ou R'_{F} un radical divalent formant un cycle respectivement avec le groupe -SO₂-C-SO₂- ou avec le groupe -SO₂-C-, ledit radical divalent étant choisi parmi les radicaux alkylènes perfluorés ayant de 2 à 12 atomes de carbone, le troisième radical présent le cas échéant étant choisi parmi les radicaux monovalents cités ci-dessus en 2) ;
4) p représente le nombre d'unités onium, et par conséquent le nombre d'anions X⁻ associés.

Comme exemples particulièrement intéressants d'anions X⁻, on peut citer les anions sulfonimidures (R_{F}SO₂NSO₂R'_{F}]⁻ dans lesquels R_{F} et R'_{F} sont choisis indépendamment l'un de l'autre dans le groupe constitué par les groupements perfluoroalkyles ayant de 1 à 10 atomes de carbone (de préférence CF₃-, C₂F₅-, C₄F₉-, C₆F₁₃-, C₈F₁₇- et C₁₀F₂₁), et ceux dans lesquels R_{F} et R'_{F} forment ensemble un radical perfluoroalkylène linéaire divalent ayant de 1 à 8 atomes de carbone.

On peut également citer les anions sulfonylméthylures [R_{F}SO₂C(Q)SO₂R'_{F}]⁻ dans lesquels Q est choisi dans le groupe constitué par les groupements alkyles, aryles, alkylaryles ou arylalkyles ayant au plus 30 atomes de carbone, les groupements perfluoroalkylsulfonyles ayant de 1 à 8 atomes de carbone (de préférence CF₃SO₂-, C₂F₅SO₂-, C₄F₉SO₂-, C₆F₁₃SO₂- et C₈F₁₇SO₂-) et les radicaux perfluoroalkyles ayant de 1 à 12 atomes de carbone (de préférence CF₃-, C₂F₅-, C₄F₉-, C₆F₁₃-, C₈F₁₇- et C₁₀F₂₁-), et R_{F} et R'_{F} sont choisis indépendamment l'un de l'autre dans le groupe constitué par les groupements alkyles ayant de 1 à 10 atomes de carbone (de préférence par CF₃-, C₂F₅-, C₄F₉-, C₆F₁₃-, C₈F₁₇-et C₁₀F₂₁-), ou R_{F} et R'_{F} forment ensemble un radical perfluoroalkylène linéaire divalent ayant de 1 à 8 atomes de carbone.

Les anions [R_{F}SO₂NSO₂R'_{F}]⁻ et [R_{F}SO₂C(SO₂R"_{F})SO₂R'_{F}] - dans lesquels R_{F}, R'_{F}, et R"_{F} représentent chacun un groupement perfluoroalkyle ayant de 1 à 10 atomes de carbone, de préférence de 1 à 6 atomes de carbone, sont particulièrement avantageux ainsi que les composés [R_{F}CH₂-O-SO₂)₂N]⁻ et [(R_{F})₂CH-O-SO₂)₂N]⁻.

Une famille particulière de composés selon l'invention comprend les sels de polyiodonium qui répondent à l'une des formules suivantes (I), (II), (III) ou (IV) : 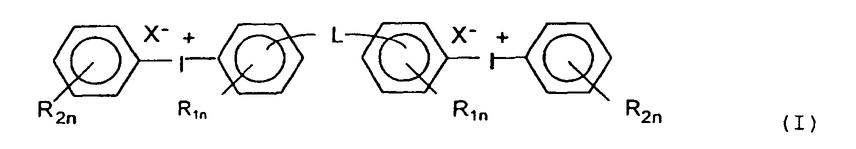 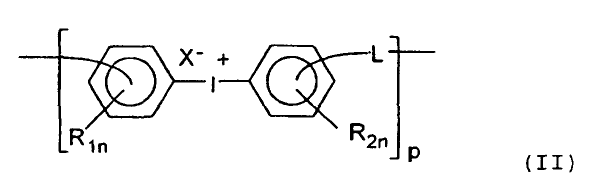 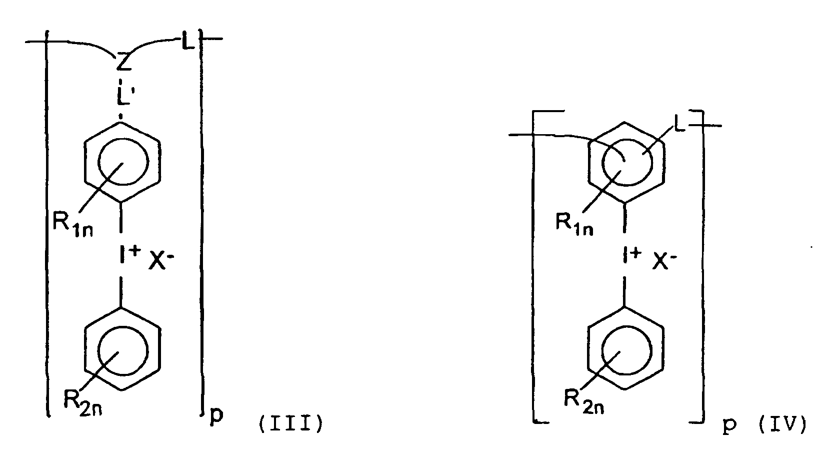 dans laquelle :
a1) R₁ₙ représente de 1 à 4, de préférence 1 à 2 groupements identiques ou différents liés à l'un quelconque des atomes de carbone libres du groupe aryle, R₂ₙ représente de 1 à 4, de préférence de 1 à 2 groupements identiques ou différents liés à l'un quelconque des atomes de carbone libres du groupe aryle, les groupements R₁ₙ et R₂ₙ étant choisis indépendamment les uns des autres parmi :
   - les radicaux alkyles ou arylalkyles linéaires ou ramifiés ayant de 1 à 30 atomes de carbone ;
   - les radicaux alkényles ayant de 1 à 30 atomes de carbone ;
   - les radicaux aryles ou alkylaryles ayant de 6 à 30 atomes de carbone, incluant ceux qui ont des noyaux condensés ;
   - les radicaux ayant de 1 à 30 atomes de carbone et choisis dans le groupe constitué par les oxaalkyles, les azaalkyles, les thiaalkyles, les phosphaalkyles, les oxaalkylènes, les azaalkylènes, les thiaalkylènes, les phosphaalkylènes,
   - les radicaux ayant de 1 à 30 atomes de carbone et incluant un groupement sulfoxyde, un groupement sulfone, un groupement oxyde de phosphine, un groupement phosphonate, tous ces radicaux étant obtenus par addition d'oxygène sur les atomes de soufre ou de phosphore ;
   - les radicaux hétérocycliques aromatiques ou alicycliques comprenant au moins un hétéroatome choisi dans le groupe constitué par O, N, S et P ;
   - -NO, -CN, -OH, -Cl, -Br, -I, -F ;
   ou bien deux substituants choisis parmi les R₁ₙ et les R₂ₙ forment ensemble un radical divalent qui forme un cycle avec le groupement qui les porte, ledit radical divalent étant choisi dans le groupe constitué par les radicaux alkylènes linéaires ayant de 1 à 18 atomes de carbone, par les biradicaux benzo portant éventuellement au moins un substituant choisi de préférence dans le groupe constitué par les radicaux alkyles, oxaalkyles ou alcényles ayant de 1 à 10 atomes de carbone, par les groupements oxaalkylènes répondant à la formule -R'-(OCH₂CH₂)_{q}-O-R'- ou -R'-[OCH(CH₃)CH₂]_{q}-O-R'- dans lesquels R' est un radical alkylène linéaire ayant de 0 à 18 atomes de carbone et 1≤q≤22;
a2) L' représente un radical divalent choisi dans le groupe constitué par les radicaux alkylènes linéaires ayant de 1 à 18 atomes de carbone, par les groupements phénylènes substitués ou non, par les groupements oxaalkylènes répondant à la formule -R'-(OCH₂CH₂)_{q}-O-R'- ou -R'-[OCH(CH₃)CH₂]_{q}-O-R'- dans lesquels R' est un radical alkylène linéaire ayant de 0 à 18 atomes de carbone et 1≤q≤22, par -O-, -S-, >C=O, par les groupements siloxanes -R'-O-[Si(R)₂O]ᵣ-R'- ou -O-[Si(R)₂O]ᵣ- 1≤r≤40 dans lesquels R' a la signification donnée ci-dessus et R est choisi dans le groupe constitué par les radicaux alkyles linéaires ayant de 1 à 18 atomes de carbone, le 2-éthylhexyle, le phényle, (de préférence R = CH₃ ou phényle) ou par une liaison directe entre deux atomes de carbone de deux groupes aryles non condensés ;
a3) L représente un radical divalent choisi dans le groupe défini au point a2) ci-dessus pour L' ; ou bien L représente un segment constitué par au moins une unité monomère non ionique ou possédant un groupement ionique non sensible à l'action d'un rayonnement actinique (L représentant dans ce cas l'espacement moyen entre les groupements ioniques actifs) ;
a4) p représente le nombre d'unités récurrentes, 2≤p≤1000
a5) Z représente -CH, -CR, -N, -SiR, -SiRO_{3,} -R étant choisi parmi les radicaux alkyles linéaires ayant de 1 à 18 atomes de carbone, le 2-éthylhexyle et le phényle ;
a6) X est tel que défini précédemment.

Parmi les composés du type polyiodonium, on préfère tout particulièrement ceux dans lesquels les substituants R₁ₙ et R₂ₙ sont choisis indépendamment les uns des autres dans le groupe constitué par les radicaux alkyles linéaires ayant de 1 à 18 atomes de carbone, le 2-éthylhexyle, le phényle, les oxaalkyles répondant à la formule R-(OCH₂CH₂)_{y}-ou R-[OCH(CH₃)CH₂]_{y}- dans lesquels R est un radical alkyle linéaire ayant de 1 à 18 atomes de carbone et 1≤y≤22.

Lorsque le composé polyiodonium de l'invention répond à l'une des formules (I) ou (II), il se présente sous la forme d'un dimère ou d'un polymère comprenant les groupements ioniques iodonium dans la chaîne du polymère.

Lorsque le composé polyiodonium répond à l'une des formules (III) ou (IV), il se présente sous la forme d'un polymère dans lequel les groupements ioniques iodonium sont portés par des substituants pendants.

Une autre famille particulière de composés selon l'invention comprend les sels de polysulfonium qui répondent à l'une des formules (V), (VI), (VII), (VIII) ou (IX) suivantes : 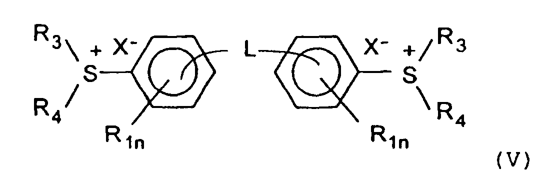 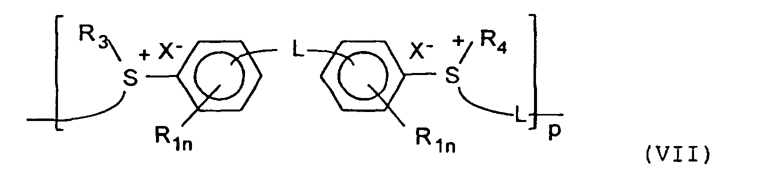 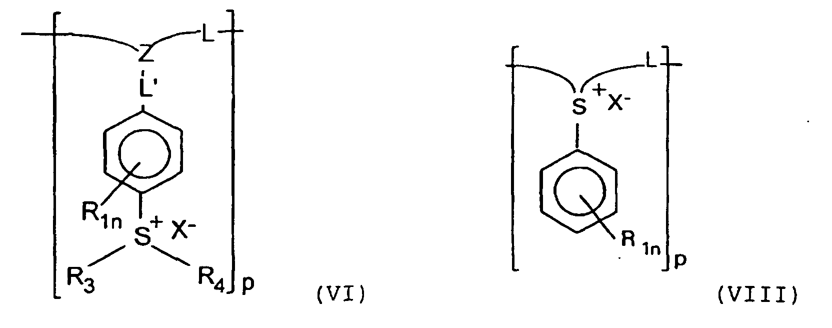 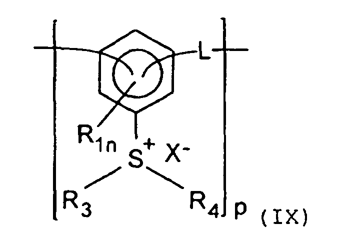 dans laquelle :
b1) R₁ₙ représente de 1 à 4, de préférence 1 à 2 groupements identiques ou différents liés à l'un quelconque des atomes de carbone libres du groupe aryle, les substituants R₁ₙ et les substituants R₃ et R₄ étant choisis indépendamment les uns des autres parmi :
   - les radicaux alkyles ou arylalkyles linéaires ou ramifiés ayant de 1 à 30 atomes de carbone ;
   - les radicaux alkényles ayant de 1 à 30 atomes de carbone ;
   - les radicaux aryles ou alkylaryles ayant de 6 à 30 atomes de carbone, incluant ceux qui ont des noyaux condensés ;
   - les radicaux ayant de 1 à 30 atomes de carbone et choisis dans le groupe constitué par les oxaalkyles, les azaalkyles, les thiaalkyles, les phosphaalkyles, les oxaalkylènes, les azaalkylènes, les thiaalkylènes, les phosphaalkylènes,
   - les radicaux ayant de 1 à 30 atomes de carbone et incluant un groupement sulfoxyde, un groupement sulfone, un groupement oxyde de phosphine, un groupement phosphonate, tous ces radicaux étant obtenus par addition d'oxygène sur les atomes de soufre ou de phosphore ;
   - les radicaux hétérocycliques aromatiques ou alicycliques comprenant au moins un hétéroatome choisi dans le groupe constitué par O, N, S et P ;
   - -NO, -CN, -OH, -Cl, -Br, -I, -F ;
   ou bien les groupements R₃ et R₄ portés par un même atome de soufre d'une part, et/ou deux substituants choisis parmi les R₁ₙ d'autre part forment ensemble un radical divalent qui forme un cycle avec le groupement qui les porte, ledit radical divalent étant choisi dans le groupe constitué par les radicaux alkylènes linéaires ayant de 1 à 18 atomes de carbone, par les biradicaux benzo portant éventuellement au moins un substituant choisi de préférence dans le groupe constitué par les radicaux alkyles, oxaalkyles ou alcényles ayant de 1 à 10 atomes de carbone, par les groupements oxaalkylènes répondant à la formule -R'-(OCH₂CH₂)_{q}-O-R'- ou -R'-[OCH(CH₃)CH₂]Q-O-R'- dans lesquels R' est un radical alkylène linéaire ayant de 0 à 18 atomes de carbone et 1≤q≤22 ;
b2) L' a la signification donnée au paragraphe a2) ci-dessus ;
b3) L a la signification donnée au paragraphe a3) ci-dessus ;
b4) p représente le nombre d'unités récurrentes, 2≤p≤1000;
b5) Z a la signification donnée au paragraphe a5) ci-dessus;
b6) X est tel que défini précédemment.

On préfère tout particulièrement les composés polysulfonium dans lesquels les substituants R₁ₙ, R₃ et R₄ sont choisis dans le groupe constitué par les radicaux alkyles linéaires ayant de 1 à 18 atomes de carbone, le 2-éthylhexyle, le phényle, les oxaalkyles répondant à la formule R-(OCH₂CH₂)_{y}- ou R-[OCH(CH₃)CH₂]_{y}- dans lesquels R est un radical alkyle linéaire ayant de 1 à 18 atomes de carbone et 1≤y≤22.

Lorsqu'un composé polysulfonium de l'invention correspondant à la formule (V) est un dimère. Un composé polysulfonium répondant à l'une des formules (VII) ou (VIII) se présente sous la forme d'un polymère portant des groupements ioniques dans la chaîne principale.

Lorsqu'un composé polysulfonium répond à l'une des formules (VI) ou (IX), il se présente sous la forme d'un polymère sur lesquels les groupements ioniques sont portés par des groupements latéraux.

Une autre famille particulière de composés selon l'invention comprend les sels de polyacylsulfonium qui répondent à l'une des formules suivantes (X), (XI), (XII), (XIII) ou (XIV) : 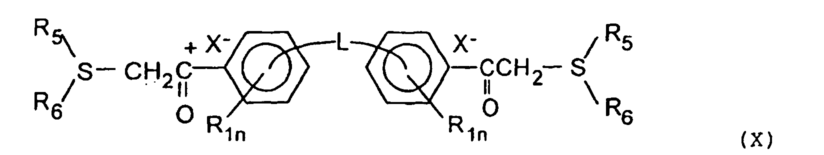 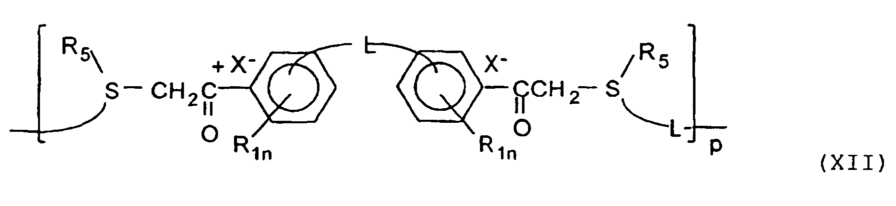 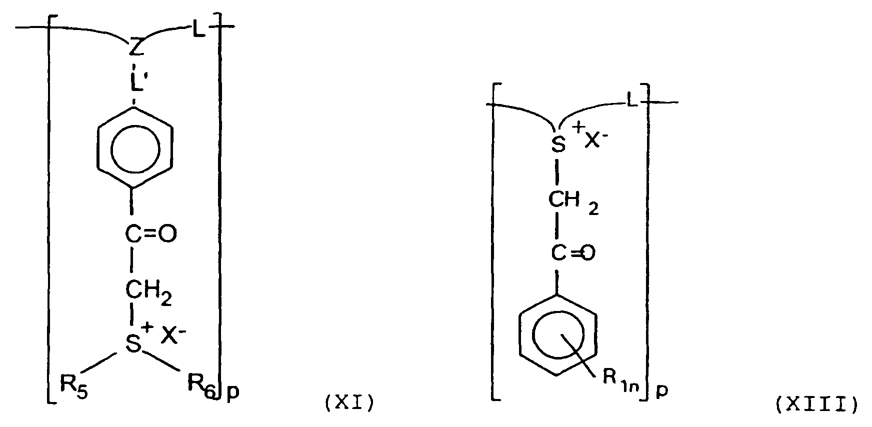 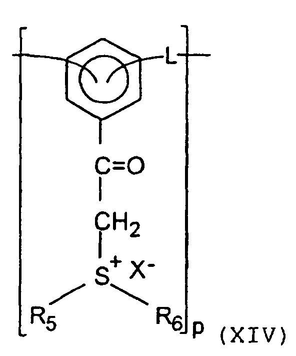 dans laquelle
c1) R₁ₙ a la signification donnée ci-dessus au paragraphe b1), et les substituants R₅ et R₆ ont la même signification que les substituants R₃ et R₄ définis ci-dessus au paragraphe b1);
c2) L' a la signification donnée au paragraphe a2) ci-dessus ;
c3) L a la signification donnée au paragraphe a3) ci-dessus ;
c4) Z a la signification donnée au paragraphe a5) ci-dessus ;
c5) p représente le nombre d'unités récurrentes, 2≤p≤1000;
c6) X est tel que défini précédemment.

Parmi les composés polyacylsulfonium, on préfère tout particulièrement ceux dans lesquels les substituants R₁ₙ, R₅ et R₆ sont choisis dans le groupe constitué par les radicaux alkyles linéaires ayant de 1 à 18 atomes de carbone, le 2-éthylhexyle, le phényle, les oxaalkyles répondant à la formule R-(OCH₂CH₂)_{y}- ou R-[OCH(CH₃)CH₂]_{y}-dans lesquels R est un radical alkyle linéaire ayant de 1 à 18 atomes de carbone et 1≤y≤22.

Une quatrième famille de composés selon l'invention comprend les sels de polydiazonium répondant à la formule (XI) 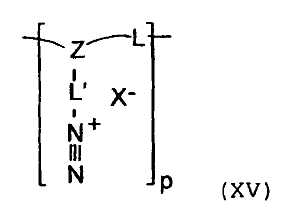 dans laquelle
d1) L a la signification donnée au paragraphe a3) ci-dessus ;
d2) L' a la signification donnée au paragraphe a2) c-dessus ;
d3) p représente le nombre d'unités récurrentes, 2≤p≤1000
d4) X représente un anion tel que défini ci-dessus.

Une cinquième famille de composés selon l'invention comprend les polyonium organométalliques répondant à l'une des formules suivantes : 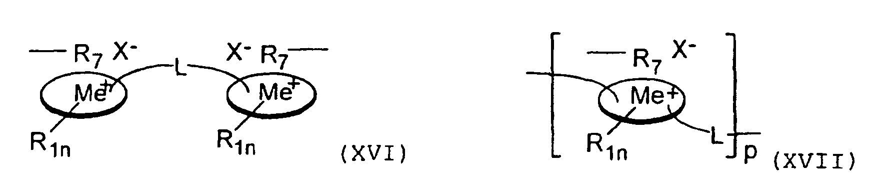 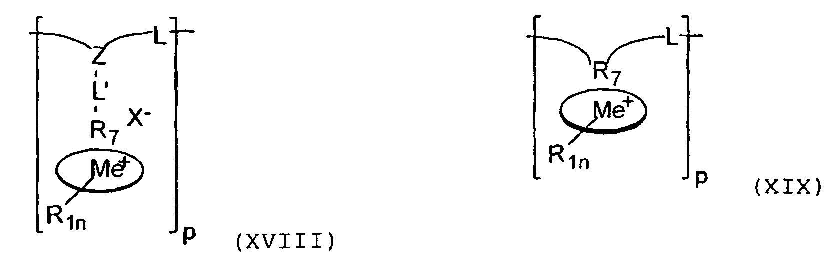 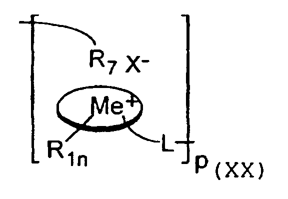 dans lesquelles
e1) R₁ₙ a la signification donnée au paragraphe a1) ci-dessus et le substituant R₇ est choisi parmi :
   - les radicaux alkyles ou arylalkyles linéaires ou ramifiés ayant de 1 à 30 atomes de carbone ;
   - les radicaux alkényles ayant de 1 à 30 atomes de carbone ;
   - les radicaux aryles ou alkylaryles ayant de 6 à 30 atomes de carbone, incluant ceux qui ont des condensés ;
   - les radicaux ayant de 1 à 30 atomes de carbone et choisis dans le groupe constitué par les oxaalkyles, les azaalkyles, les thiaalkyles, les phosphaalkyles, les oxaalkylènes, les azaalkylènes, les thiaalkylènes, les phosphaalkylènes,
   - les radicaux ayant de 1 à 30 atomes de carbone et incluant un groupement sulfoxyde, un groupement sulfone, un groupement oxyde de phosphine, un groupement phosphonate, tous ces radicaux étant obtenus par addition d'oxygène sur les atomes de soufre ou de phosphore ;
   - les radicaux hétérocycliques aromatiques ou alicycliques comprenant au moins un hétéroatome choisi dans le groupe constitué par O, N, S et P ;
   - -NO, -CN, -OH, -Cl, -Br, -I, -F ;
e2) L' a la signification donnée au paragraphe a2) ci-dessus ;
e3) L a la signification donnée au paragraphe a3) ci-dessus ;
e4) p représente le nombre d'unités récurrentes, 2≤p≤1000;
e5) Z à la signification donnée au paragraphe a5) ci-dessus
e6) X étant un anion tel que défini ci-dessus ;
e7) Me représente un métal de transition choisi dans le groupe des élements de transition des colonnes 3 à 12 (lignes 3 à 6) de la classification périodique.

Comme exemples de composés dans lesquels la partie cationique est un polycation organométallique, on peut citer les polymères contenant les unités ferrocénium, (en particulier ceux qui incorporent l'unité vinylférrocène, les unités polyalkylbenzène-fer-cyclopentadiène), les polymères qui incorporent des unités nickelocènium, et les polymères qui incorporent des unités tricarbonyl manganèsecyclopentadiène.

Les composés (A⁺X⁻)ₚ de la présente invention sont en général insolubles dans l'eau. Ils peuvent donc être préparés par un procédé consistant à effectuer une métathèse dans l'eau ou un mélange eau/alcool léger (méthanol, éthanol, propanol) entre composé (A⁺X₁⁻)ₚ qui est un sel soluble du polycation (A⁺)ₚ dans lequel l'anion X₁⁻ a un caractère hydrophile, et un composé soluble dans l'eau de l'anion X- ayant un cation fortement hydrophile.

Les sels solubles (A⁺X₁⁻)ₚ du polycation (A⁺)ₚ sont choisis de préférence parmi les sels dans lesquels l'anion X₁⁻ est choisi parmi un hydroxyde, un chlorure, un bromure, un hydrogénosulfate, un dihydrogénophosphate ou un méthylsulfonate. Ces anions étant fortement solvatés par l'eau ou les alcools légers, ils favorisent la solubilité.

Les composés solubles dans l'eau ou les mélanges eau/alcools de l'anion X⁻ sont choisis de préférence parmi les perhaloalkylsulfonimides et les perhaloalkylsulfonyl-méthanes, les perhaloalkylsulfonimidures et les perhalo-alkylsulfonylméthylures de lithium, de sodium, de potassium, d'ammonium, de calcium ou de magnésium. Le choix du cation dépend bien entendu de la facilité d'obtention et du minimum d'hydrophilie requise pour entraîner la solubilité.

Lorsque le composé polyionique (A⁺X⁻)ₚ de l'invention est préparé à partir d'un sel (A⁺X₁⁻)ₚ dans lequel X₁ est un chlorure, un bromure, un alkylsulfonate, un alkyloxy-sulfonate ou un arylsulfonate, dont les sels de Na ou de K sont insolubles dans les solvant usuels, il est avantageux de conduire la réaction en présence d'un sel de Na ou K de l'anions X⁻. La solubilité de ces sels dans les solvants même de moyenne polarité étant appréciable, les sels insolubles tel que NaCl, Kbr précipitent alors que le composé polyionique de l'invention reste en solution. Comme solvants, on peut citer l'acétone, le méthyléthylcétone, l'acétonitrile, le THF, les esters tels que les formiates ou l'acétate de méthyle ou d'éthyle.

Il faut noter que tout autre procédé d'échange ionique peut être mis en oeuvre, par exemple un procédé utilisant une résine échangeuse d'ions, ou un procédé de précipitation sélective.

De manière surprenante, il est apparu que les composés polyioniques de l'invention sont solubles dans la plupart des solvants organiques usuels, contrairement à des composés polyioniques de l'art antérieur tels que les polystyrène-iodoniumn les poly thiaphénylsulfonium malgré une densité de charge élévée. Ces derniers ne sont solubles que dans l'eau et les solvants très polaires tels que la diméthylformamide (DMF) ou le carbonate de propylène suivant le type de contre ion. Or l'eau est incompatible, avec les réactions de polymérisation cationique et n'est pas un solvant de la plupart des monomères. Les solvants tels la DMF ou le carbonate de propylène sont très difficiles à éliminer du fait de leur température d'ébullition élevée et ils peuvent ultérieurement contaminer les matières en contact avec les produits de la polymérisation/réticulation (industrie alimentaire, sérigraphies...). Les polycations de l'invention ont dès lors pu être utilisés comme photoamorceurs pour des réactions de polymérisation cationique. Par rapport aux composés monomères ioniques, ils présentent un avantage certain : lorsqu'ils sont utilisés comme photoamorceurs, ils se décomposent sous l'action du rayonnement actinique sans laisser de résidus suceptibles de migrer dans la phase polymérique obtenue.

La présente invention a par conséquent pour objet l'utilisation des composés polyioniques (A⁺X⁻)ₚ de l'invention comme photoamorceurs sources d'acides de Brønsted catalyseurs de polymérisation ou de réticulation de monomères ou de prépolymères capables de réagir par voie cationique, ou catalyseurs pour la modification des paramètres de solubilité de polymères. Le procédé de polymérisation ou de réticulation de monomères ou de prépolymères capables de réagir par voie cationique est caractérisé en ce que l'on utilise un composé de l'invention comme photoamorceur source d'acide catalysant la réaction de polymérisation.

Lorsque le composé polyionique de l'invention est destiné à être utilisé comme photoamorceur pour la polymérisation de monomères ou de prépolymères polymérisant par voie cationique, le choix des groupements R₁ à R₈ est effectué parmi les radicaux ci-dessus pour augmenter la solubilité dudit composé dans les solvants de mise en oeuvre des monomères ou des prépolymères et en fonction des propriétés souhaitées pour le polymère final. Par exemple, le choix de radicaux alkyles non substitués donne une solubilité dans les milieux peu polaires. Le choix de radicaux comprenant un groupe oxa ou une sulfone donnera une solubilité dans les milieux polaires. Les radicaux incluant un groupement sulfoxyde, un groupement sulfone, un groupement oxyde de phosphine, un groupement phosphonate obtenu par addition d'oxygène sur les atomes de soufre ou de phosphore peuvent conférer au polymère obtenu des propriétés améliorées en ce qui concerne l'adhésion, la brillance, la résistance à l'oxydation ou aux UV.

Les monomères et les prépolymères qui peuvent être polymérisés ou réticulés à l'aide des photoamorceurs polyioniques de la présente invention sont ceux qui peuvent subir une polymérisation cationique.

Parmi les monomères qui peuvent être polymérisés ou réticulés à l'aide d'un composé polyionique de l'invention utilisé comme photoamorceur, on peut citer les monomères qui comportent une fonction éther cyclique, une fonction thioéther cyclique ou une fonction amine cyclique les composés vinyliques, (plus particulièrement les éthers vinyliques), les oxazolines, les lactones et les lactames.

Parmi les monomères du type éther ou thioéther cyclique, on peut citer l'oxyde d'éthylène, l'oxyde de propylène, l'oxétane, l'épichlorhydrine, le tétrahydrofurane, l'oxyde de styrène, l'oxyde de cyclohexène, l'oxyde de vinylcyclohexène, le glycidol, l'oxyde de butylène, l'oxyde d'octylène, les éthers et les esters de glycidyle (par exemple le méthacrylate ou l'acrylate de glycidyle, le phényl glycidyl éther, le diglycidyléther de bisphénol A ou ses dérivés fluorés), les acétals cycliques ayant de 4 à 15 atomes de carbone (par exemple le dioxolane, le 1,3-dioxane, le 1,3-dioxépane).

Parmi les composés vinyliques, les éthers vinyliques constituent une famille très importante de monomères sensibles en polymérisation cationique. A titre d'exemple, on peut citer l'éthyl vinyl éther, le propyl vinyl éther, l'isobutyl vinyl éther, l'octadécyl vinyl éther, l'éthylèneglycol monovinyl éther, le diéthylèneglycol divinyl éther, le butanediol monovinyl éther, le butanediol divinyl éther, l'hexanediol divinyl éther, l'éthylèneglycol butyl vinyl éther, le triéthylèneglycol méthyl vinyl éther, le cyclohexanediméthano monovinyl éther, le cyclohexane-diméthanol divinyl éther, le 2-éthylhexyl vinyl éther, le poly-THF-divinyl éther ayant une masse comprise entre 150 et 5000, le diéthylèneglycol monovinyl éther, le triméthylolpropane trivinyl éther, l'aminopropyl vinyl éther, le 2-diéthylaminoéthyl vinyl éther.

On peut également citer les éthers méthyl-vinyliques qui contiennent un ou plusieurs groupements CH3CH=CH-O- et qui sont avantageusement obtenus par isomérisation des éthers allyliques correspondants en présence d'un catalyseur tel que (Φ₃P)2RuCl₂.

Comme autres composés vinyliques qui peuvent être soumis à une polymérisation cationique en présence d'un composé polyionique de l'invention utilisé en tant que photoamorceur, on peut citer à titre d'exemple les 1,1-dialkyléthylènes (par exemple l'isobutène), les monomères aromatiques vinyliques (par exemple le styrène, les α-alkylstyrène, notamment l'α-méthylstyrène, le 4-vinylanisole, l'acénaphtène), les composés N-vinyliques (par exemple la N-vinylpyrolidone, le N-vinyl formamide ou les N-vinyl sulfonamides).

Parmi les prépolymères, on peut citer les composés dans lesquels des groupements époxy sont portés par une chaîne aliphatique, une chaîne aromatique, ou une chaîne hétérocyclique, par exemple les éthers glycidiques du bisphénol A éthoxylés par 3 à 15 unités d'oxyde d'éthylène ou de propylène, les siloxanes possédant des groupements latéraux du type époxycyclohexène-éthyle obtenus par hydrosilylation des copolymères de di alkyl, d'alkylaryle ou de diaryl siloxane avec le méthyl hydrogénosiloxane en présence d'oxyde de vinylcyclohexène, ou d'éthers insaturés difonctionnels possédant une extrémité vinyléther et une extrémité méthylvinyléther (propènyl), les produits de condensation du type sol-gel obtenus à partir du triéthoxy ou du triméthoxy silapropylcyclohexène oxyde, les uréthanes incorporant les produits de réaction du butanediol monovinyléther et d'un alcool de fonctionnalité supérieure ou égale à 2 sur un di ou un tri isocyanate aliphatique ou aromatique.

Le procédé de polymérisation selon l'invention consiste à mélanger au moins un monomère ou prépolymère capable de polymériser par voie cationique et au moins un composé polyionique (A⁺X⁻)ₚ de l'invention, et à soumettre le mélange obtenu à un rayonnement actinique ou un rayonnement β. De préférence, le mélange réactionnel est soumis au rayonnement après avoir été mis sous forme d'une couche mince ayant une épaisseur inférieure à 5 mm, de préférence sous forme d'un film mince ayant une épaisseur inférieure ou égale à 500 *µ*m. La durée de la réaction dépend de l'épaisseur de l'échantillon et de la puissance de la source à la longueur d'onde λ active. Elle est définie par la vitesse de défilement devant la source, qui est comprise entre 300 m/min et 1 cm/min. Des couches de matériau final ayant une épaisseur supérieure à 5 mm peuvent être obtenues en répétant plusieurs fois l'opération consistant à épandre une couche et à la traiter par le rayonnement.

Généralement, la quantité de photoamorceur polyionique (A⁺X⁻)ₚ utilisé est comprise entre 0,01 et 15 % en poids par rapport au poids de monomère ou de prépolymère, de préférence entre 0,1 et 5 % en poids.

Un composé polyionique (A⁺X⁻)ₚ de la présente invention peut être utilisé comme photoamorceur en l'absence de solvant, notamment lorsque l'on souhaite polymériser des monomères liquides dans lesquels le sel est soluble ou aisément dispersable. Cette forme d'utilisation est particulièrement intéressante, car elle permet de supprimer les problèmes liés aux solvants (toxicité, inflammabilité).

Un composé polyionique (A⁺X⁻)ₚ de la présente invention peut également être utilisé en tant que photoamorceur sous forme d'une solution homogène dans un solvant inerte vis-à-vis de la polymérisation, prête à l'emploi et aisément dispersable, en particulier dans le cas où le milieu à polymériser ou à réticuler présente une viscosité élevée.

Comme exemple de solvant inerte, on peut citer les solvants volatils, tels que l'acétone, la méthyl-éthyl cétone et l'acétonitrile. Ces solvants serviront simplement à diluer les produits à polymériser ou à réticuler (pour les rendre moins visqueux, surtout lorsqu'il s'agit d'un prépolymère). Ils seront éliminés après la polymérisation ou la réticulation par séchage. On peut également citer les solvants non volatils. Un solvant non volatil sert de la même manière qu'un solvant volatil, à diluer les produits que l'on veut polymériser ou réticuler, et à dissoudre le composé polyionique) (A⁺X⁻)ₚ de l'invention utilisé comme photoamorceur, mais en outre il restera dans le matériau formé et il agira ainsi comme plastifiant. A titre d'exemple, on peut citer le carbonate de propylène, la γ-butyrolactone, les éther-esters des mono-, di-, triéthylène ou propylène glycols, les éther-alcools des mono-, di-, tri- éthylène ou propylène glycols, les plastifiants tels que les esters de l'acide phtalique ou de l'acide citrique.

Dans un autre mode de mise en oeuvre de l'invention, on utilise comme solvant ou diluant un composé réactif vis-à-vis de la polymérisation, qui est un composé de faible masse moléculaire et de faible viscosité qui va jouer à la fois le rôle de monomère polymérisable et le rôle de solvant ou de diluant pour des monomères plus visqueux ou des prépolymères utilisés conjointement. Après la réaction, ces monomères ayant servi de solvant font partie du réseau macromoléculaire finalement obtenu, leur intégration étant plus grande lorsqu'il s'agit de monomères bi-fonctionnels. Le matériau obtenu après irradiation ne contient plus de produits ayant un faible poids moléculaire et une tension de vapeur appréciable, ou susceptibles de contaminer les objets avec lesquels le polymère est en contact. A titre d'exemple, un solvant réactif peut être choisi parmi les mono et di éthers vinyliques des mono-, di-, tri-, tétra-éthylène et propylène glycols, la N-méthylpyrolidone, le 2-propényléther du carbonate de propylène commercialisé par exemple sous la dénomination PEPC par la société ISP, New Jersey, Etats-Unis.

Pour irradier le mélange réactionnel, le rayonnement peut être choisi parmi le rayonnement ultraviolet, le rayonnement visible, les rayons X, les rayons γ et le rayonnement β. Lorsque l'on utilise la lumière ultraviolette comme rayonnement actinique, il peut être avantageux d'ajouter aux photoamorceurs de l'invention des photosensibilisateurs destinés à permettre une photolyse efficace avec les longueurs d'ondes moins énergétiques que celles correspondant au maximum d'absorption du photoamorceur, telles que celles émises par les dispositifs industriels, (λ ≈ 300 nm pour les lampes à vapeur de mercure en particulier). De tels additifs sont connus, et à titre d'exemples non limitatifs, on peut citer l'anthracène, le diphényl-9,10-anthracène, le pérylène, la phénothiazine, le tétracène, la xanthone, la thioxanthone, l'acétophénone, la benzophénone, les 1,3,5-triaryl-2-pyrazolines et leurs dérivés, en particulier les dérivés de substitution sur les noyaux aromatiques par des radicaux alkyles, oxa- ou aza-alkyles permettant entre autre de changer la longueur d'onde d'absorption. L'isopropylthioxantone est un exemple de photosensibilisateur préféré lorsque l'on utilise un composé polyiodonium A^{P+}pX⁻ selon l'invention comme photoamorceur.

Parmi les différents types de rayonnement mentionnés, le rayonnement ultraviolet est particulière préféré. D'une part, il est plus commode d'emploi que les autres rayonnements mentionnés. D'autre part, les photoamorceurs sont en général directement sensibles aux rayons UV et les photosensibilisateurs sont d'autant plus efficaces que la différence d'énergie (δλ) est plus faible.

Les composés polyioniques (A⁺X⁻)ₚ de l'invention peuvent aussi être mis en oeuvre en association avec des amorceurs de type radicalaire générés thermiquement ou par action d'une radiation actinique. Il est ainsi possible de polymériser ou de réticuler des mélanges de monomères ou de prépolymères contenant des fonctions dont les modes de polymérisation sont différents, par exemple des monomères ou des prépolymères polymérisant par voie radicalaire et des monomères ou des prépolymères polymérisant par voie cationique. Cette possibilité est particulièrement avantageuse pour créer des réseaux interpénétrés ayant des propriétés physiques différentes de celles qui seraient obtenues par simple mélange des polymères issus des monomères correspondants. Les éthers vinyliques ne sont pas ou sont peu actifs par amorçage radicalaire. Il est donc possible, dans un mélange réactionnel contenant un photoamorceur selon l'invention, un amorceur radicalaire, au moins un monomère du type éther vinylique et au moins un monomère comprenant des doubles liaisons non activées telles que celles des groupes allyliques, d'effectuer une polymérisation séparée de chaque type de monomère. Il est par contre connu que les monomères déficients en électrons, tels que les esters ou les amides de l'acide fumarique, de l'acide maléique, de l'acide acrylique ou méthacrylique, de l'acide itaconique, de l'acrylonitrile, du méthacrylonitrile, la maléimide et ses dérivés, forment en présence d'éthers vinyliques riches en électrons, des complexes de transfert de charge donnant des polymères alternés 1:1 par amorçage radicalaire. Un excès initial de monomères vinyliques par rapport à cette stoechiométrie permet de préserver des fonctions polymérisables par initiation cationique pure. Le déclenchement de l'activité d'un mélange d'amorceur radicalaire et d'amorceur cationique selon l'invention peut être fait simultanément pour les deux réactifs dans le cas par exemple d'insolation par un rayonnement actinique d'une longueur d'onde pour laquelle les photoamorceurs de l'invention et les amorceurs radicalaires choisis sont actifs, par exemple à λ = 250 nm. A titre d'exemple, on peut citer comme amorceurs les produits commerciaux suivantes : Irgacure 184®, Irgacure 651®, Irgacure 261® Quantacure DMB®, Quantacure ITX®.

Il peut aussi être avantageux d'utiliser les deux modes de polymérisation d'une manière séquentielle, pour former dans un premier temps des prépolymères dont la mise en forme est aisée et dont le durcissement, l'adhésion, la solubilité ainsi que le degré de réticulation peuvent être modifiés par le déclenchement de l'activité de l'amorceur cationique. Par exemple, un mélange d'un amorceur radicalaire thermodissociable et d'un photoamorceur cationique selon l'invention permet de réaliser des polymérisations ou des réticulations séquentielles, d'abord sous l'action de la chaleur, puis sous l'action d'un rayonnement actinique. D'une manière similaire, si l'on choisit un amorceur radicalaire et un photoamorceur cationique selon l'invention, le premier étant photosensible à des longueurs d'ondes plus longues que celle déclenchant le photoamorceur selon l'invention, on obtient une réticulation en deux étapes contrôlables. Des amorceurs radicalaires peuvent être par exemple Irgacure® 651 permettant d'amorcer des polymérisations radicalaires à des longueurs d'onde de 365 nm.

L'invention a également pour objet l'utilisation des composés polyioniques (A⁺X⁻)ₚ de l'invention pour les réactions d'amplification chimique de photoresists pour la microlithographie. Lors d'une telle utilisation, un film d'un matériau comprenant un polymère et un composé polyionique (A⁺X⁻)ₚ de l'invention est soumis à une irradiation. L'irradiation provoque la formation de l'acide HX, qui catalyse la décomposition ou la transformation du polymère. Après décomposition ou transformation du polymère sur les parties du film qui ont été irradiées, les monomères formés ou le polymère transformé sont éliminés et il reste une image des parties non exposées. Pour cette application particulière, il est avantageux d'utiliser des composés polymères comprenant des unités vinyles portant un substituant ionique. Parmi ces composés, on peut citer les sels de polyiodonium répondant à la formule (III), les sels de polysulfonium répondant à la formule (VI), les polyacylsulfonium répondant à la formule (XI), les polydiazonium répondant à la formule (XV), les sels de complexes organométalliques répondant à la formule (XVIII). Ces composés permettent d'obtenir après photolyse des produits qui ne sont pas volatils, et donc pas odorants lorsqu'il s'agit de sulfures. Parmi les composés de l'invention, on préfère tout particulièrement les polysulfonium qui sont particulièrement efficaces comme photoamorceur, les phénacylsulfonium et les polymères et copolymères de vinyl ferrocénium qui peuvent être obtenus facilement. Parmi les polymères qui peuvent ainsi être modifiés en présence d'un composé de l'invention, on peut citer notamment les polymères contenant des motifs ester ou des motifs aryléther d'alcool tertiaire, par exemple les poly(phtalaldéhydes), les polymères de bisphénol A et d'un diacide, le polytertiobutoxycarbonyl oxystyrène, le polytertiobutoxy-a-méthyl styrène, le polyditertiobutyl-fumarate-co-allyltriméthylsilane et les polyacrylates d'un alcool tertiaire, en particulier le polyacrylate de tertiobutyle. D'autres polymères sont décrits dans J.V. Crivello et al, Chemistry of Materials 8, 376-381, (1996).

Les composés polyioniques (A⁺X⁻)ₚ de la présente invention, qui présentent une grande stabilité thermique, offrent de nombreux avantages par rapport aux sels connus de l'art antérieur. Ils ont des vitesses d'amorçage et de propagation comparables ou supérieures à celles obtenues à l'aide des anions de coordination de type PF₆⁻, AsF₆⁻ et surtout SbF₆⁻. Les sels M⁺X⁻ dans lesquels M est un métal alcalin et X un anion identique à l'anion d'un composé de la présente invention sont connus pour être facilement solvatés dans les polymères, dont ils augmentent peu la température de transition vitreuse. En outre, le coefficient de diffusion de l'anion X⁻ est supérieur à celui des anions hexafluorométallates ou des anions tétrafluoroborates ou des anions phénylborates. Ces propriétés sont expliquées par la délocalisation de la charge négative et la flexibilité de l'anion autour des liaisons SNS ou SCS.

Un autre avantage intéressant des composés polyioniques (A⁺X⁻)ₚ de l'invention est l'absence d'éléments toxiques tels que P, As ou Sb, ces éléments étant par ailleurs considérés comme des agents contaminants dans les procédés de la micro-électronique.

Il faut en outre mentionner la possibilité de moduler les paramètres de solubilité et les températures de fusion des sels de l'invention par le choix des groupements R_{F}, R'_{F} ainsi que Q tels que définis ci-dessus, adaptés à la polarité du milieu d'utilisation pour les réactions de polymérisation, de réticulation ou d'amplification chimique. La très faible tension superficielle caractéristique des groupements perfluorés confère aux composés polyioniques (A⁺X⁻)ₚ de l'invention des propriétés tensioactives utiles pour le mélange et le dispersion homogène de pigments et charges dans les mélanges polymérisables.

La présente invention est décrite ci-après plus en détail, par les exemples suivants qui sont donnés à titre d'illustration, l'invention n'étant toutefois pas limitée à ces exemples.

### Exemple 1

### Préparation d'un sel de poly(iodonium)

On a préparé un composé comprenant des groupements iodophényles en transformant les noyaux aromatiques d'un échantillon de polystyrène (M_{w} 6000) par iodation du polystyrène, oxydation en iodosoacétate par le mélange acide acétique / anhydride acétique / peroxyde d'hydrogène selon la méthode décrite par Yamada et al. (*Die Makromolecular Chemie,* (1972), 152, 153-162). 10 g du composé ainsi préparé ont été mis en suspension dans un mélange de 30 ml d'acide méthanesulfonique et 5 ml de butoxybenzène maintenu à 0°C pendant 4 heures. Le produit de réaction a été versé dans 400 ml d'un mélange 2:1 v/v éther : isopropanol et le précipité a été séparé par filtration, lavé par du THF et séché. 8 g du méthanesulfonate de polyiodonium ainsi obtenu ont été mis en suspension dans 50 ml d'eau et on a ajouté 10 g de sel de lithium du bis(nonafluorobutanesulfonyl)imidure de lithium en solution dans 25 ml d'eau. Le mélange a été agité pendant 1 heure et séparé par filtration. Le rendement de l'échange ionique (métathèse) est quantitatif pour l'obtention de bis(nonafluorobutanesulfonyl)imidure de poly(vinylphényl-(4-butoxyphényl)-4-iodonium. La structure du polymère est :

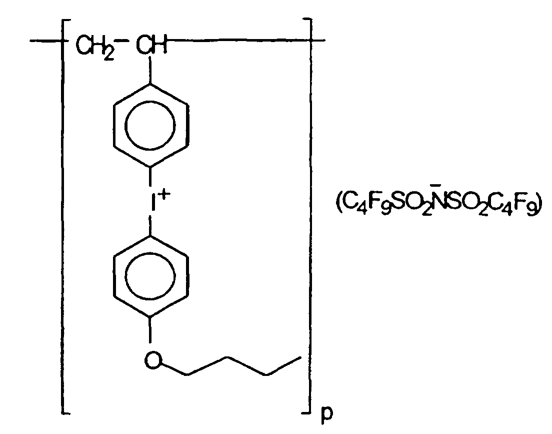

### Exemple 2

### Préparation d'un sel de poly(sulfonium) à partir d'un sel de polyiodonium

3 g du composé polyionique de l'exemple 1 ont été mélangés avec 5 ml de 4-butylphénylthioéther et 500 mg de benzoate de cuivre finement dispersés, et chauffés à 130° pendant trois heures en phase fondue. Après réaction, le mélange a été dissous dans 30 ml d'acétone et précipité dans 100 ml d'éther, à trois reprises, puis il a été traité dans le système acétone (solvant) / eau (précipitant).

On a obtenu le sel de polysulfonium :

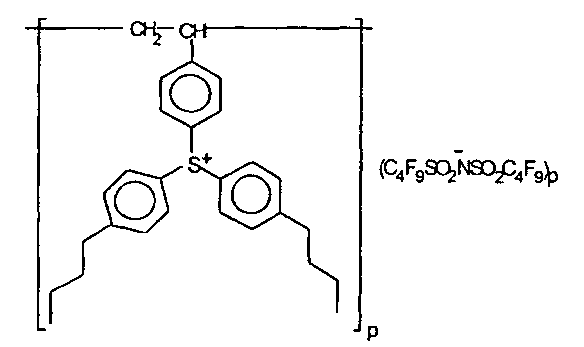

Les paramètres de solubilité des deux composés polymères ioniques obtenus dans les exemples 1 et 2 sont résumés dans le tableau suivant :

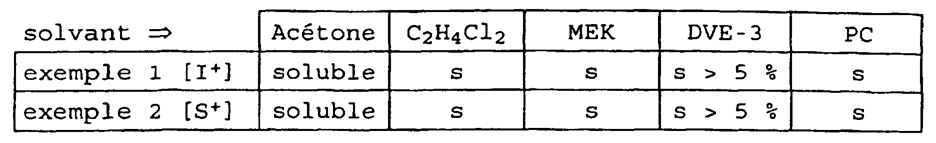

- s =: solubilité > 20% en poids
- DVE-3 =: triéthylène glycol divinyl éther
- MEK =: méthyléthylcétone
- PC =: carbonate de propylène

Le caractère exceptionnel de solubilité de polyélectrolytes possédant une densité de charge élevée est ainsi mis en évidence.

### Exemple 3

On a préparé un polymère similaire à celui de l'exemple 1 mais avec un groupement méthoxy au lieu de butoxy, par action du poly(4-iodostyrène) (23 g) sur l'anisole (10,8 g) en présence d'acide peracétique (45,6 g) et d'acide toluènesulfonique (19,05 g) dans le dichlorométhane (40 ml) en suivant le mode opératoire de Crivello & Lee, (US. 4,780,511). On a dispersé 25 g du polymère obtenu dans un mélange 1:1 d'éthanol et d'acétone, auquel on a ajouté 15 g de sel de sodium de la bis (trifuorométhanesulfonyl)imide. La solution visqueuse obtenue a été centrifugée pour éliminer le toluènesulfonate de sodium, puis précipitée par l'eau. On a ainsi obtenu une poudre jaune soluble dans les solvants usuels.

### Exemple 4

On a préparé le phénoxyéthylvinyléther en faisant réagir le chloroéthylvinyléther sur le phénate de sodium, puis on a effectué une polymérisation dans le dichlorométhane initiée par TiCl₄ à -10°C. Par réaction de 8,2 g du polymère ainsi obtenu sur 9,8 g de phényliodosotoluène sulfonate dans 40 ml d'un mélange équivolumique d'acide acétique et de dichlorométhane, on a obtenu un polyiodonium qui a été précipité par l'éther. La réaction d'échange ionique a ensuite été conduite de la même manière que dans l'exemple 3 entre 12 g de polymère et 7 g de sel de sodium de la bis(trifluorométhanesulfonyl)imide. Après précipitation et lavage à l'eau, on a obtenu une masse collante qui peut être utilisée en solution à 50% dans la méthyléthylcétone.

### Exemple 5

Un oligomère contenant des groupements iodonium a été préparé selon la méthode décrite dans *J. Polym. Sci., Polym. Lett.* (1976), 14, 65 sous forme de bromure, par action de 5,7 g de 1,4-bis(diacétoxyiodo)phénylène sur 20 ml de 1,3-diphénylpropane dans 300 ml d'anhydride acétique et 40 ml d'acide sulfurique à 0°C. Le mélange a été versé dans 1 l d'eau contenant 80 g de bromure de sodium et 100 g d'acétate de sodium. Le précipité a été filtré, lavé à l'éther et séché. 4 g de cet oligomère ont été mis en solution dans 50 ml d'eau et on a ajouté 5 g de tris(trifluorométhanesulfonyl)méthylure de sodium dissous dans 25 ml d'eau. Le tris(trifluorométhane-sulfonyl)méthylure de polyiodonium a précipité et a été séparé par filtration. Il est soluble dans les solvants polaires usuels comme l'acétone, ou dans des monomères comme le (4-propényloxymethylène)-1,3dioxolane-2one (PEPC commercialisé par International Specialty Products, Wayne, NJ, USA) . Il faut noter qu'un bromure d'un cation polyiodonium identique ne possède qu'une solubilité négligeable dans les solvants les plus polaires connus, tels que le diméthylsulfoxyde (DMSO), le diméthylformamide (DMF), ou l'hexaméthylphosphoramide (HMPA).

### Exemple 6

Les propriétés de solubilité de certains composés polyioniques de la présente invention ont été comparées aux propriétés d'un poly(iodonium) préparé selon Crivello & Lam (*J. Polym. Sci.; Polymer Chemistry,* (1979), 17, 3845-3858). Le polyiodonium selon Crivello et al a été obtenu par une trans-addition du chlorure de (4,4'-N-maléimido)diphényliodonium avec le 1,10-décanethiol dans le m-crésol en présence d'une base tertiaire, suivie d'une métathèse en présence de NaPF₆. Les composés de la présente invention ont été préparés de la même manière, mais en remplaçant pour la métathèse NaPF₆ par l'un des sels suivants : Na⁺(CF₃SO₂)₂N⁻, Na⁺(CF₃SO₂)N(SO₂C₄F₉)⁻, Na⁺(CF₃SO₂)₃C⁻.

Les résultats sont groupés dans le tableau suivant :

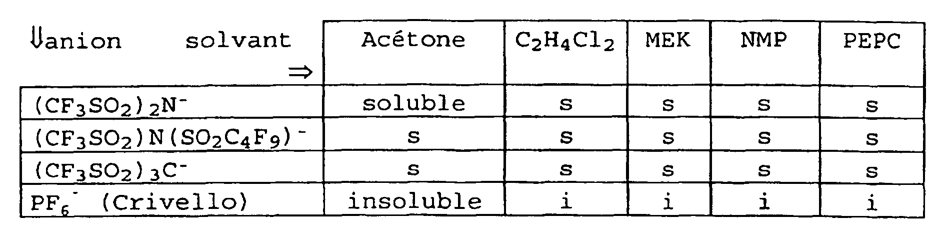

- s =: solubilité > 20% en poids
- i =: insoluble
- MEK =: méthyléthylcétone
- PEPC =: carbonate de propylène + carbonate d'éthylène
- NMP =: N-méthylpyrrolidone

### Exemple 7

Un polythioéther a été préparé par réaction de 15 g de dimercaptohexane avec 18,67 g de 1,2 bis(2-chloroéthoxy) éthane dans 200 ml de N-méthylpyrrolidone à 150°C en présence de 10 g de carbonate de potassium. Le polymère obtenu a été précipité dans l'eau et purifié par plusieurs opérations de dissolution (CH₂Cl₂) / précipitation (diéthyléther), puis séparé par centrifugation dans un récipient unique. Le polymère se présente sous la forme d'une masse collante.

On a ensuite introduit 7g du polymère dissous dans 120 ml de dichlorométhane (CH₂Cl₂) dans un ballon tricol équipé d'un réfrigérant, d'une agitation mécanique et d'une entrée de gaz neutre (argon), et on a ajouté goutte à goutte 4,8 g de 2-bromoacétophénone. Le mélange a été chauffé au reflux 40°C. Un précipité est apparu rapidement. La réaction a été poursuivie 12 heures après l'apparition du précipité. Le bromure de polyphénacylsulfonium a été séparé par filtration et lavé avec du dichlorométhane et de l'éther. 5 g du composé polyionique ont été dissous dans 60 ml d'eau, la solution a été filtrée, puis on a ajouté sous agitation 3 g de sel de lithium de bis(trifluorométhanesulfonyl)imide (Li[CF₃SO₂]₂N) en solution dans 25 ml d'eau. Un précipité s'est formé immédiatement et l'agitation a été poursuivie pendant 1 h. Le polymère a été séparé par filtration et séché. Le polymère a été obtenu avec un rendement quantitatif.

Les propriétés de solubilité du composé polyionique obtenu sont résumées dans le tableau suivant:

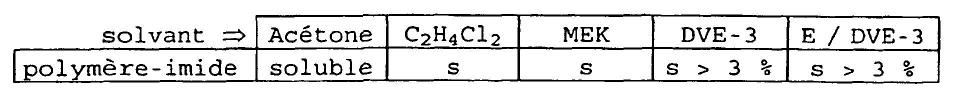

- s =: solubilité > 20 % en poids
- DVE-3 =: triéthylène glycol divinyl éther
- MEK =: méthyléthylcétone
- E =: Epoxyde (bisphénol A diglycidyléther).

### Exemple 8

Un polythioéther similaire à celui de l'exemple précédent a été préparé par réaction de 8 g de 2-mercapto-éthyléther avec 15,3 g de α,α-dibromo-*m*-xylène dans 100 ml de diméthylformamide à 80°C en présence de 4 g de carbonate de potassium. Le polymère a été précipité dans l'eau et purifié comme précédemment. Dans un ballon tricol équipé d'un réfrigérant, d'une agitation mécanique et d'une entrée de gaz neutre (Argon), 10 g du polymère ont été dissous dans 120 ml d'hexane et on a ajouté goutte à goutte 11,5 g de bromoacétyl-4-octyloxybenzène). Le mélange a été chauffé au reflux à 60°C. Un précipité est apparu rapidement. La réaction a été poursuivie 12 heures après l'apparition du précipité. Le bromure de polyphénacylsulfonium a été séparé par filtration et lavé avec de l'hexane et de l'éther. 7 g du composé polyionique ont été mis en suspension dans 60 ml d'acétone et 5,15 g de sel de potassium du tris(trifluorométhanesulfonyl)méthane (K[CF₃SO₂]₃C) ont été ajoutés sous agitation. Le mélange réactionnel a été maintenu sous agitation pendant 1 heure à température ambiante, puis la solution a été filtrée pour éliminer le bromure de potassium formé. L'acétone a été évaporée et on a obtenu une masse très visqueuse du composé polyionique.

Les propriétés de solubilité du composé polyionique obtenu sont indiquées dans le tableau suivant :

| | |
|---|---|
| solvant | |
| Acétone | ∞ |
| C₂H₄Cl₂ | ∞ |
| MEK | ∞ |
| DVE-3 | s > 10 % |
| époxy / VGE | s > 10 % |
| toluène | s |
| BVE-1 | s |
| photo-1 | c > 1% |
| PDMS 10 | s > 2 % |
| PDMS 500 | s > 1 % |

- ∞=: solubilité totale
- c =: compatible (absence de micro-séparation de phase visible optiquement)
- MEK =: méthyléthylcétone
- E =: bisphénol A diglycidyléther.
- DVE-3 =: triéthylène glycol divinyl éther
- BVE-1 =: butanediolmonovinyléther
- photo-1 =: résine pour résist à amplification chimique : *poly(t-butoxycarboxystyrène-co-*cyanoéthylacrylate) 1:1
- PDMS 10 =: α,ω-triméthylsiloxy-polydiméthylsiloxane; viscosité 10 cSt, M_{w} Å 1250
- PDMS 500 =: dito ; viscosité 500 cSt, M Å 17250
- E / VGE =: 50 v/v bisphénol A diglycidyléther, triéthylène glycol divinyl éther

### Exemple 9

6,5 g de trifluorométhanesulfonamide CF₃SO₂NH₂ et 10,8 ml de pyridine dans 60 ml de dichlorométhane ont été refroidis à -15°C et on a ajouté goutte à goutte 3,6 ml de chlorure de sulfuryle dans 10 ml de dichlorométhane, puis 4,6 ml de 1,1,1,3,3,3-hexafluoro-2-propanol. Le mélange a été agité pendant 1 heure à -15°C, puis 4 heures à température ambiante (25°C). Le mélange réactionnel a ensuite été filtré et le solvant a été éliminé à l'aide d'un évaporateur rotatif. Le résidu solide obtenu a été dissous dans 50 ml d'eau contenant 5 g d'acétate de sodium, et 18,45 g de bis(hexafluorophosphate de bis[4-(diphénylsulfonio)phényl]thioéther préparé selon la méthode d'Akhtar et al. (*Chem. Mat.* (1990), 2, 732 et K.T. Chang, US 4,197,174) et finement pulvérisé ont été ajoutés. Le mélange a été introduit dans un flacon de polyéthylène rigide contenant des boulets d'oxyde de zirconium et agité à la température de la pièce pendant 3 h dans un broyeur à rouleaux. La suspension obtenue a été filtrée et le solide obtenu a été séché. On a ainsi obtenu 28 g (rendement = 78%) du composé de structure suivante :

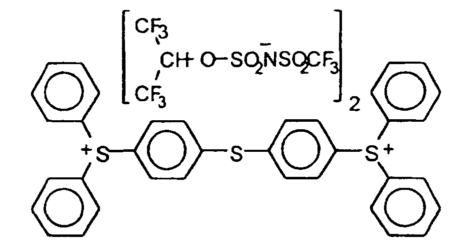

### Exemple 10

A 4 g de dimère cyclopentadiényl-fer-dicarbonyle (commercialisé par Aldrich, Milwaukee, USA) dissous dans 20 ml d'acétonitrile et maintenu à 0°C, on a ajouté (en maintenant une forte agitation) 0,58 ml de brome dilué dans 5 ml d'acétonitrile ; la réaction de formation du sel CpFe(CO)2Br a été immédiate et quantitative. 20 g de 2-isopropényl-4-cumène (p-isopropyl-styrène) ont été polymérisés dans 200 ml de dichlorométhane à -10°C par amorçage cationique initié par 150 µl de SnCl₄. Le polymère a été précipité dans l'éther et purifié par plusieurs opérations de dissolution (CH₂Cl₂) / précipitation (diéthyléther). On a obtenu le polymère sous forme d'un solide blanc, avec un rendement de 63%. Dans un ballon tricol équipé d'un réfrigérant, d'une agitation mécanique et d'une entrée de gaz neutre (Argon), 8,2 g du polymère ont été dissous dans 120 ml de dichloroéthane (C₂H₄Cl₂) et on a ajouté la solution de CpFe(CO)₂Br dans l'acétonitrile préparée précédemment. Le mélange a été chauffé au reflux. Un précipité s'est formé avec dégagement concomitant de monoxyde de carbone. La réaction a été poursuivie 2 heures après l'apparition du précipité. Le bromure de poly(arèneferrocènium) a été séparé par filtration et lavé avec du dichlorométhane et de l'acétonitrile. 3 g du composé polyionique ont été dissous dans 50 ml d'eau auxquels ont été ajoutés 3,8 g de sel de lithium de (nonafluorobutanesulfonyl)-(trifluorométhylsulfonyl)imide (Li[C₄F₉SO₂NSO₂CF₃]) dans 25 ml d'eau ; un précipité s'est formé immédiatement et l'agitation a été poursuivie pendant 1 h. Le polymère a été séparé par filtration et séché. Il a été obtenu avec un rendement quantitatif.

Un autre composé polyionique a été préparé de la même manière, mais en remplaçant le sel de lithium de (nonafluorobutanesulfonyl)-(trifluorométhylsulfonyl)-imide par 1,5 g d'hexafluorophosphate de sodium. Les propriétés de solubilité des deux composés polyioniques obtenus sont résumées à titre indicatif dans le tableau suivant:

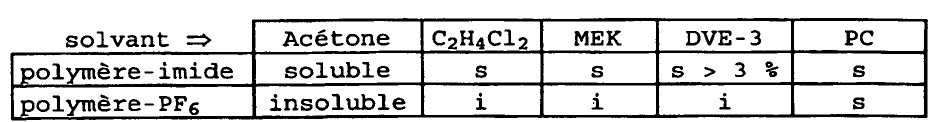

- DVE3 =: triéthylène glycol divinyl éther
- PC =: propylène carbonate
- s =: solubilité > 20% en poids
- i =: insoluble
- MEK =: méthyléthylcétone

### Exemple 11

A 5 g de 1,2-diferrocenyléthane (commercialisé par Aldrich C°, Milwaukee, USA) en solution dans 50 ml de toluène on a ajouté 3,4 g de [bis(trifluoro-acétoxy)iodo)benzène. Un précipité bleu qui s'est formé immédiatement, a été séparé, lavé à l'éther et séché. 5 g de ce solide ont été mis en solution dans 25 ml d'eau et on y a ajouté 4,9 g de bis(trifluorométhanesulfonyl)imidure de sodium. On a obtenu précipité de sel de l'anion imidure du dimère du ferrocène sous forme d'une poudre cristalline bleue, avec un rendement quantitatif.

### Exemple 12

On a ajouté 3,3 g de [bis(trifluoroacétoxy)-iodo]benzène (commercialisé par Aldrich) à 6 g d'un copolymère de vinylferrocène (commercialisé par Aldrich C°, Milwaukee, USA) et de méthacrylate de butyle contenant 42% de motifs organométalliques obtenu par polymérisation radicalaire induite par l'azobis(butyronitrile) en solution dans le toluène). Un précipité bleu s'est formé immédiatement et il a été séparé, puis lavé à l'éther pour éliminer l'excès d'oxydant, et séché. 5g de ce composé polyionique (dans lequel le polycation de ferricinium est associé à l'anion trifluoroacétate) ont été mis en suspension dans 25 ml d'eau à laquelle ont été ajoutés 3 g de bis(trifluorométhanesulfonyl)imidure de sodium. Le précipité du composé polyionique de l'anion imidure du polyferricinium a été obtenu avec un rendement quantitatif sous forme d'une poudre amorphe bleue soluble dans la plupart des solvants usuels.

### Exemple 13

Dans un ballon tricol équipé d'un réfrigérant, d'une agitation mécanique et d'une entrée de gaz neutre (Argon), 9,5 g d'un copolymère de diméthylsiloxane et d'(hydrogéno) (méthyl)-siloxane (HMS 301 25% SiH, M_{w} 1900, commercialisé par Gelest Inc., Tullytown, PA, USA) ont été mis en solution dans le THF. On a ajouté 7,9 g d'allylferrocène et 70 mg d'acide chloroplatinique H₂PtCl₆. Le mélange a été chauffé au reflux pendant 4 heures. Un prélèvement a permis de confirmer la disparition complète des bandes IR de la liaison SiH. Le polymère a été précipité dans le méthanol et purifié par trois opérations de dissolution (THF) / précipitation (méthanol). A 5,4 g de ce polymère en solution dans le dichlorométhane, on a ajouté 2,4 g de [bis(trifluoroacétoxy)iodo]benzène. Le produit de réaction a été versé dans 100 ml d'éther et le précipité a été séparé par centrifugation. Une métathèse permettant de remplacer l'anion trifluoroacétate par l'anion tris(trifluorométhanesulfonyl) méthylure a été effectuée dans l'eau (100 ml) entre 4,2 g du poly(cation) et 3,1 g du sel de sodium tris(trifluorométhanesulfonyl)méthylure de sodium dissous dans 25 ml d'eau. Le polymère a été séparé par centrifugation et se présente sous le forme d'une masse gommeuse bleue soluble dans la plupart de solvant, y compris à des teneurs > 3% dans les huiles siliconées. La structure du polymère est la suivante :

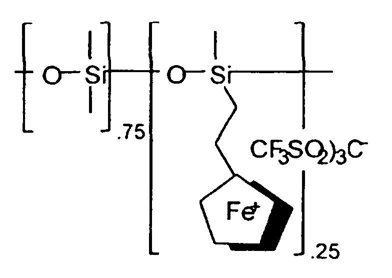

### Exemple 14

Un oligomère de diazonium a été préparé selon la méthode décrite dans U.S. 2,714,066 par condensation du chlorozincate de 4-diazodiphénylamine avec la formaldéhyde. 25 g de ce composé polyionique ont été dissous dans 500 ml d'eau maintenue à 0°C et contenant 50 g d'acétate de sodium et 28 g de di-sel de sodium de l'acide éthylène diamine tétraacétique (EDTA). On a ajouté alors 24 g de sel de sodium de la bis(trifluorométhanesulfonyl)imide en solution dans 50 ml d'eau. Le composé polyionique a été séparé par filtration et séché (rendement quantitatif), et conservé à 0°C à l'abri de la lumière. Ce composé est très soluble dans les solvants organiques usuels moyennement polaires tels que la MEK, et soluble dans les monomères de type DVE-3 ou PEPC, ou les éthers glycidiques.

### Exemple 15

### Préparation d'un photoresist négatif

A 2 g de poly(4-hydroxystyrène)-co-styrène (8:2) (commercialisé par Shinetsu, Japon) en solution dans 20 ml de diméthylformamide, ont été ajoutés 9,7 ml d'une solution 1M d'hydroxyde de potassium dans le méthanol et 1,3 g de chloroéthylvinyléther. La solution a été chauffée à 80°C pendant 1 heure et le mélange réactionnel a été versé dans 100 ml d'eau, où le poly(4-vinyloxyéthyl)-styrène formé a précipité. Le polymère a été purifié par plusieurs opérations de dissolution / précipitation dans l'acétone (solvant) / eau (précipitant) et acétone (solvant) / éther (précipitant). 1 g de poly(4-vinyloxy-éthyl)-styrène-co-styrène et 20 mg du polymère de l'exemple 1 dans 10 ml de MEK ont été déposés à la tournette (spin-coated) sur un substrat de silicium de manière à former un film de 0,5 µm d'épaisseur. Le film a été soumis à une exposition de 1 MJ/cm² obtenue par un laser KrF à travers un masque interférentiel. Le développement a été effectué par le THF. La résolution obtenue, observée par microscope électronique (SEM), est de l'ordre de l'épaisseur du film, soit 0,5 µm. Ce photorésist ne contient aucun élément métallique susceptible de contaminer le silicium.

### Exemple 16

### Photorésist positif

1g de poly(4-t-butoxycarboxystyrène) dans le dichloroéthane et 60 mg du polymère de l'exemple 8 ont été déposés à la tournette (spin-coated) sur un substrat de silicium de manière à former un film de 0,5 µm d'épaisseur. Le film a ensuite été soumis à une exposition de 1 MJ/cm² obtenue par un laser KrF à travers un masque interférentiel. Le développement a été effectué par une solution à 4% d'hydroxyde de tétraméthyl ammonium dans l'eau. La résolution obtenue, observée par microscope électronique (SEM), est de l'ordre de l'épaisseur du film, soit 0,5 µm. Ce photorésist ne contient aucun élément métallique susceptible de contaminer le silicium.

### Exemple 17

Les propriétés de photo-amorçage des composés polyioniques de l'invention sont illustrées sur le tableau suivant.

Les composés polyioniques des exemples précédents ont été utilisés à raison de 1% en poids dans différents monomères et irradiés par un rayonnement U.V. à 254 nm avec une puissance de 1900 mW/cm² durant une durée de 5 secondes, suivie d'une période de 10 minutes permettant la propagation des espèces générées dans le milieu (postcure). Les résultats sont indiqués dans le tableau ci-après.

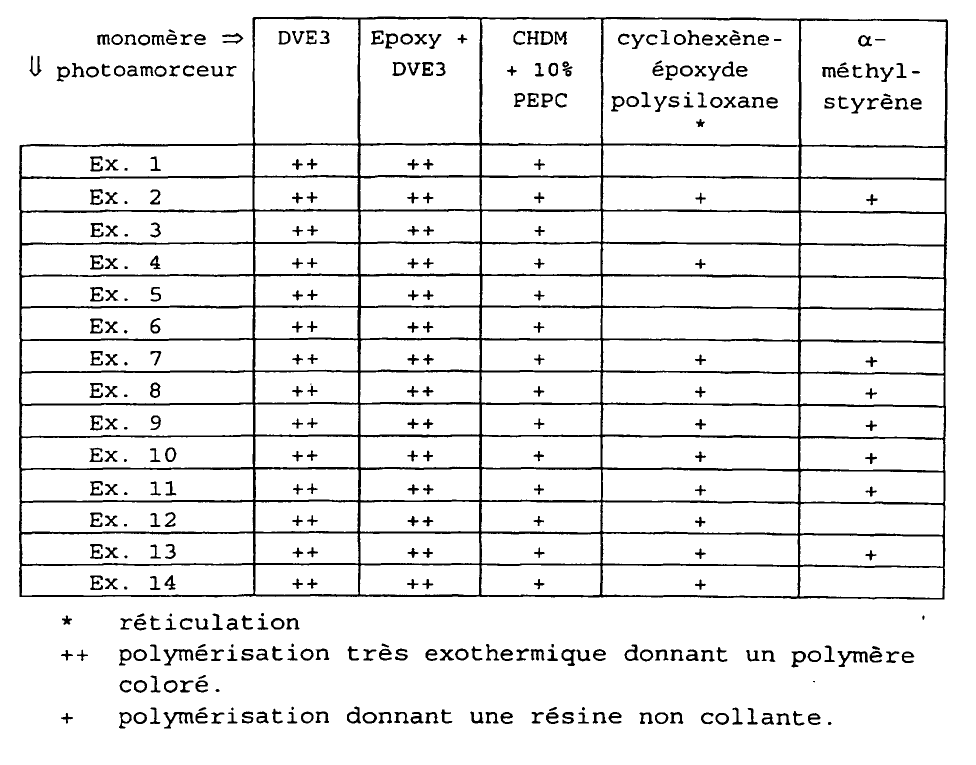

## Revendications

1. Composé ionique polymère ou oligomère constitué par une partie polycationique (A⁺)ₚ comprenant plusieurs unités onium et un nombre d'anions X⁻ suffisant pour assurer la neutralité électrique du composé, **caractérisé en ce que** :
- les unités onium sont choisies dans le groupe constitué par les biaryliodonium, les arylsulfonium, les arylacylsulfonium, les diazonium, les cations organométalliques comprenant un métal de transition complexé par au moins un cycle insaturé comprenant de 4 à 12 atomes de carbone ;
- X⁻ est un anion imidure [R_{F}SO₂NSO₂R'_{F}]⁻ ou un anion méthylure [R_{F}SO₂C(Q)SO₂R'_{F}]⁻ dans lesquels :
1) Q représente :
- H-, Cl-, F-, Br- ou CN- ;
- un radical alkyle ayant de 1 à 30 atomes de carbone ;
- un radical aryle ou alkylaryle ou arylalkyle ayant de 6 à 30 atomes de carbone ;
- un groupe R"_{F} ou un groupe R"_{F}SO₂ ;
2) R_{F} et R'_{F}, ainsi que R"_{F} le cas échéant lorsque X⁻ est un anion méthylure, sont choisis indépendamment l'un de l'autre dans le groupe constitué par le fluor, les groupements perhaloalkyles ayant de 1 à 30 atomes de carbone, les groupements (perhaloalkyl)alkyloxy, les groupements cycloaliphatiques perhalogénés ayant de 3 à 30 atomes de carbone contenant éventuellement des hétéroatomes choisis parmi O et N, et/ou portant éventuellement au moins un chaînon perhaloalkyle, les groupements aryles perhalogénés ayant de 6 à 30 atomes de carbone ; ou bien
3) R_{F} et R'_{F} forment ensemble un radical divalent formant un cycle respectivement avec le groupe -SO₂-N-SO₂- ou avec le groupe -SO₂-C(Q)-SO₂-, ou bien, lorsque X⁻ est un anion méthylure, R"_{F} forme avec l'un des radicaux R_{F} ou R'_{F} un radical divalent formant un cycle respectivement avec le groupe -SO₂-C-SO₂- ou avec le groupe -SO₂-C-, ledit radical divalent étant choisi parmi les radicaux alkylènes perfluorés ayant de 2 à 12 atomes de carbone, le troisième radical présent le cas échéant étant choisi parmi les radicaux monovalents cités ci-dessus en 2) ;
4) p représente le nombre d'unités onium.

2. Polymère ionique selon la revendication 1, **caractérisé en ce que** l'anion est un sulfonimidure [R_{F}SO₂NSO₂R'_{F}]⁻ ou un sulfonylméthylure [R_{F}SO₂C(Q)SO₂R'_{F}]⁻ dans lesquels :
- Q est choisi dans le groupe constitué par les groupements alkyles, aryles, alkylaryles ou arylalkyles ayant au plus 30 atomes de carbone, les groupements perfluoroalkylsulfonyles ayant de 1 à 8 atomes de carbone et les radicaux perfluoroalkyles ayant de 1 à 12 atomes de carbone ;
- R_{F} et R'_{F} sont choisis indépendamment l'un de l'autre dans le groupe constitué par les groupements perfluoroalkyles ayant de 1 à 10 atomes de carbone, ou bien ;
- R_{F} et R'_{F} forment ensemble un radical perfluoroalkylène linéaire divalent ayant de 1 à 8 atomes de carbone.

3. Polymère ionique selon la revendication 1, constitué par un sel de polyiodonium répondant à l'une des formules (I) à (IV) suivantes, ou par un sel de polysulfonium répondant à l'une des formules (V) à (IX) suivantes, ou par un sel de polyacylsulfonium répondant à l'une des formules (X) à (XIV) suivantes, ou par un sel de polydiazonium répondant à la formule (XV) suivante, ou par un sel de polyonium organométallique, répondant à l'une des formules (XVI) à (XX) suivantes : 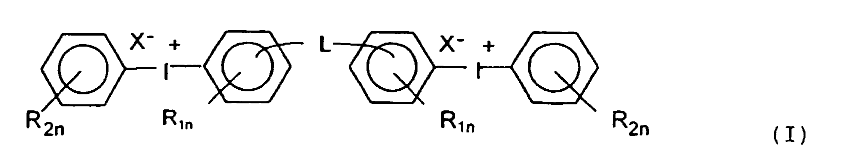 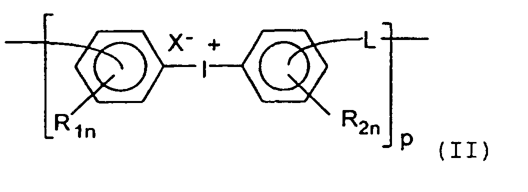 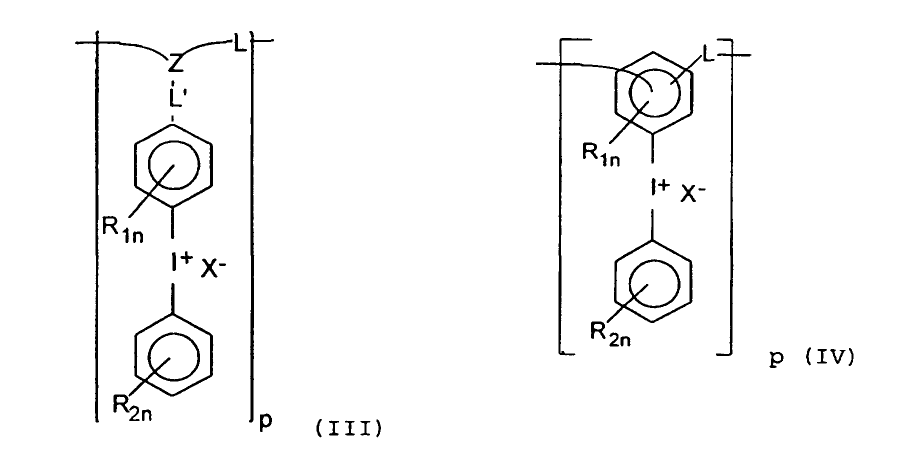 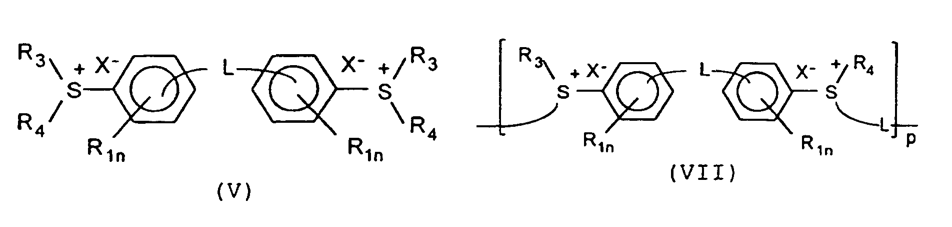 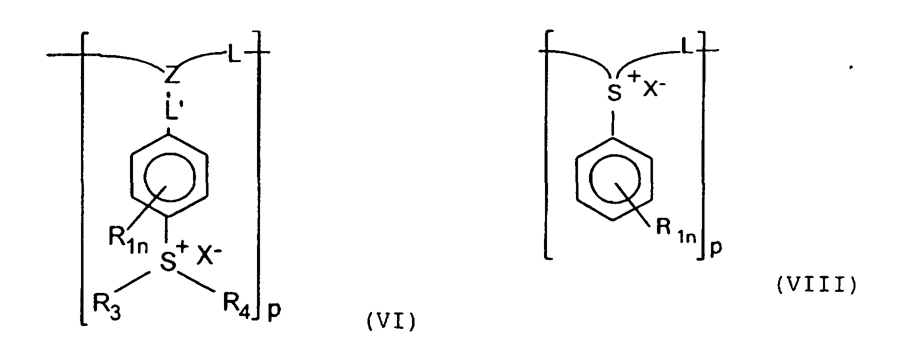 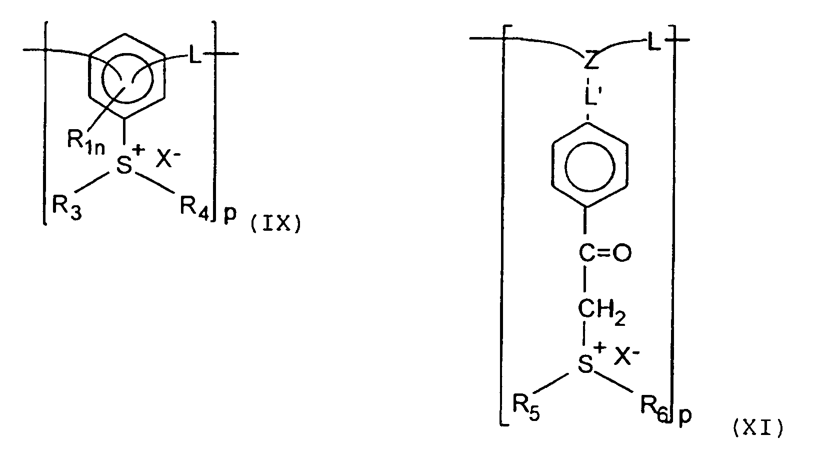 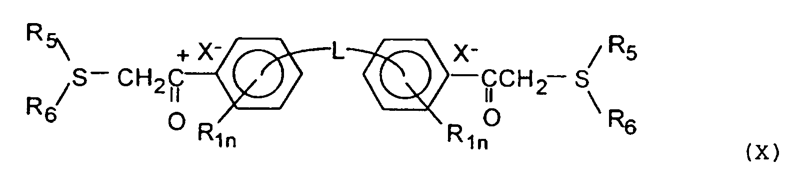 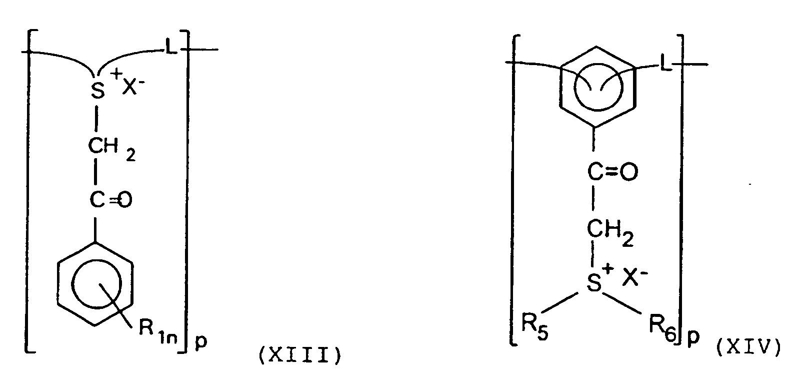 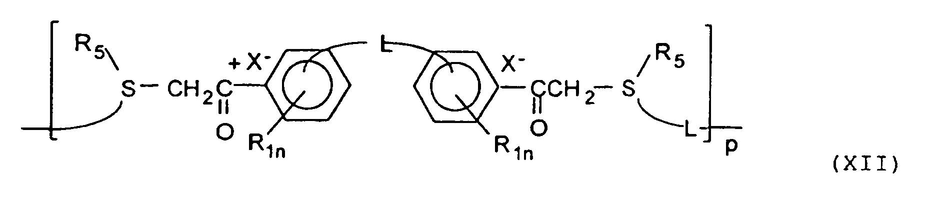 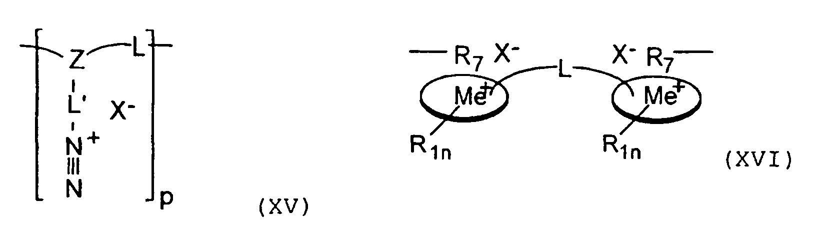 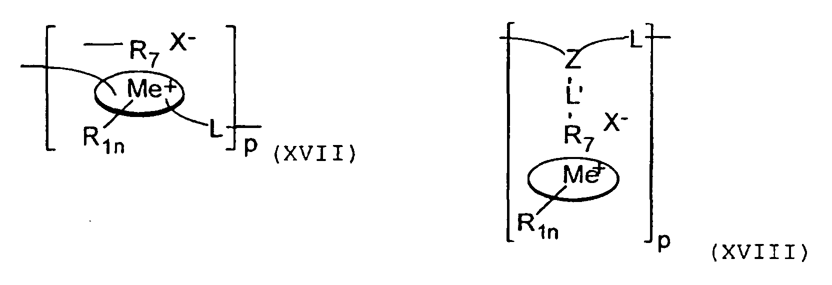 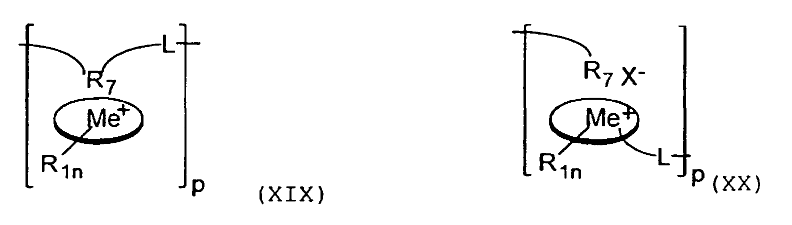 dans lesquelles :
a1) R₁ₙ représente de 1 à 4, de préférence 1 à 2 groupements identiques ou différents liés à l'un quelconque des atomes de carbone libres du groupe aryle, R₂ₙ représente de 1 à 4, de préférence de 1 à 2 groupements identiques ou différents liés à l'un quelconque des atomes de carbone libres du groupe aryle, les groupements R₁ₙ et R₂ₙ ainsi que les groupements R₃ à R₈ sont choisis indépendamment les uns des autres parmi :
- les radicaux alkyles ou arylalkyles linéaires ou ramifiés ayant de 1 à 30 atomes de carbone ;
- les radicaux alkényles ayant de 1 à 30 atomes de carbone ;
- les radicaux aryles ou alkylaryles ayant de 6 à 30 atomes de carbone, incluant ceux qui ont des noyaux condensés ;
- les radicaux ayant de 1 à 30 atomes de carbone et choisis dans le groupe constitué par les oxaalkyles, les azaalkyles, les thiaalkyles, les phosphaalkyles, les oxaalkylènes, les azaalkylènes, les thiaalkylènes, les phosphaalkylènes,
- les radicaux ayant de 1 à 30 atomes de carbone et incluant un groupement sulfoxyde, un groupement sulfone, un groupement oxyde de phosphine, un groupement phosphonate, tous ces radicaux étant obtenus par addition d'oxygène sur les atomes de soufre ou de phosphore ;
- les radicaux hétérocycliques aromatiques ou alicycliques comprenant au moins un hétéroatome choisi dans le groupe constitué par O, N, S et P ;
- -NO, -CN, -OH, -Cl, -Br, -I, -F ;
ou bien deux substituants choisis parmi les R₁ₙ et les R₂ₙ et/ou les substituants R₃ et R₄ et/ou les substituants R₅ et R₆ forment ensemble un radical divalent qui forme un cycle avec le groupement qui les porte, ledit radical divalent étant choisi dans le groupe constitué par les radicaux alkylènes linéaires ayant de 1 à 18 atomes de carbone, par les biradicaux benzo portant éventuellement au moins un substituant choisi de préférence dans le groupe constitué par les radicaux alkyles, oxaalkyles ou alcényles ayant de 1 à 10 atomes de carbone, par les groupements oxaalkylènes répondant à la formule -R'-(OCH₂CH₂)_{q}-O-R'- ou -R'-[OCH(CH₃)CH₂]_{q}-O-R'- dans lesquels R' est un radical alkylène linéaire ayant de 0 à 18 atomes de carbone et 1≤q≤22;
a2) L' représente un radical divalent choisi dans le groupe constitué par les radicaux alkylènes linéaires ayant de 1 à 18 atomes de carbone, par les groupements phénylènes substitués ou non, par les groupements oxaalkylènes répondant à la formule -R'-(OCH₂CH₂)_{q}-O-R'- ou -R'-[OCH(CH₃)CH₂]_{q}-O-R' - dans lesquels R' est un radical alkylène linéaire ayant de 0 à 18 atomes de carbone et 1≤q≤22, par -O-, -S-, >C=O, par les groupements siloxanes -R'-O-[Si(R)₂O]ᵣ-R'- ou -O-[Si(R)₂O]ᵣ- 1≤r≤40 dans lesquels R' a la signification donnée ci-dessus et R est choisi dans le groupe constitué par les radicaux alkyles linéaires ayant de 1 à 18 atomes de carbone, le 2-éthylhexyle, le phényle, (de préférence R = CH₃ ou phényle) ou par une liaison directe entre deux atomes de carbone de deux groupes aryles non condensés ;
a3) L représente un radical divalent choisi dans le groupe défini au point a2) ci-dessus pour L' ; ou bien L représente un segment constitué par au moins une unité monomère non ionique ou possédant un groupement ionique non sensible à l'action d'un rayonnement actinique (L représentant dans ce cas l'espacement moyen entre les groupements ioniques actifs) ;
a4) p représente le nombre d'unités récurrentes, 2≤p≤1000
a5) Z représente CH, CR, N, SiR, SiRO₃, R étant choisi parmi les radicaux alkyles linéaires ayant de 1 à 18 atomes de carbone, le 2-éthylhexyle et le phényle ;
a6) Me représente un métal de transition choisi dans le groupe des élements de transition des colonnes 3 à 12 (lignes 3 à 6) de la classification périodique.

4. Procédé de préparation d'un polymère ionique selon la revendication 1, **caractérisé en ce qu'**il consiste à effectuer une métathèse dans l'eau ou dans un mélange eau/alcool léger entre un sel (A⁺X₁⁻)ₚ du polycation (A⁺)ₚ et un composé A₁⁺X⁻ solubles dans le milieu réactionnel, l'anion X₁ ayant un caractère hydrophile, et le cation A₁⁺ étant choisi parmi les métaux alcalins et les métaux alcalion-terreux.

5. Procédé selon la revendication 4, **caractérisé en ce que** l'anion X₁⁻ est un hydroxyde, un chlorure, un bromure, un hydrogénosulfate, un dihydrogénophosphate ou un méthylsulfonate.

6. Procédé de polymérisation ou de réticulation de monomères ou de prépolymères capables de réagir par voie cationique, **caractérisé en ce que** l'on utilise un composé selon la revendication 1 comme photoamorceur source d'acide catalysant la réaction.

7. Procédé selon la revendication 6, **caractérisé en ce que** les monomères sont choisis dans le groupe constitué par les composés qui comportent une fonction éther cyclique, une fonction thioéther cyclique ou une fonction amine cyclique, les composés vinyliques, les éthers vinyliques, les oxazolines, les lactones et les lactames.

8. Procédé selon la revendication 6, **caractérisé en ce que** le prépolymère est choisi dans le groupe constitué par les composés dans lesquels des groupements époxy sont portés par une chaîne aliphatique, une chaîne aromatique, ou une chaîne hérérocyclique.

9. Procédé selon la revendication 6, **caractérisé en ce qu'**il consiste à mélanger le photoamorceur avec au moins un monomère ou prépolymère capable de polymériser par voie cationique, et à soumettre le mélange obtenu à un rayonnement actinique ou un rayonnement β.

10. Procédé selon la revendication 9, **caractérisé en ce que** le mélange réactionnel est soumis au rayonnement après avoir été mis sous forme d'une couche mince.

11. Procédé selon la revendication 6, **caractérisé en ce que** la quantité de photoamorceur utilisé est comprise entre 0,01 et 15 % en poids par rapport au poids de monomère ou de prépolymère.

12. Procédé selon la revendication 6, **caractérisé en ce que** le photoamorceur est utilisé sous forme d'une solution dans un solvant inerte vis à vis de la réaction de polymérisation.

13. Procédé selon la revendication 12, **caractérisé en ce que** le solvant inerte est choisi dans le groupe constitué par l'acétone, la méthyl-éthyl cétone, l'acétonitrile, le carbonate de propylène, la γ-butyrolactone, les éther-esters des mono-, di-, triéthylène ou propylène glycols, les éther-alcools des mono-, di-, tri- éthylène ou propylène glycols, les esters de l'acide phtalique ou de l'acide citrique.

14. Procédé selon la revendication 6, **caractérisé en ce que** la réaction est effectuée en présence d'un solvant ou d'un diluant constitué par un composé réactif vis à vis de la polymérisation.

15. Procédé selon la revendication 14, **caractérisé en ce que** le composé réactif est choisi dans le groupe constitué par les mono et di éthers vinyliques des mono-, di-, tri-, tétra- éthylène ou propylène glycols, le trivinyl éther triméthylolpropane et le divinyléther du diméthanol-cyclohexane, la N-vinylpyrolidone, le 2-propényléther du carbonate de propylène.

16. Procédé selon la revendication 6, **caractérisé en ce que** l'on ajoute un photosensibilisateur au milieu réactionnel.

17. Procédé selon la revendication 16, **caractérisé en ce que** le photosensibilisateur est choisi dans le groupe constitué par l'anthracène, le diphényl-9,10-anthracène, le pérylène, la phénothiazine, le tétracène, la xanthone, la thioxanthone, l'isopropylthioxantone, l'acétophénone, la benzophénone, les 1,3,5-triaryl-2-pyrazolines et leurs dérivés, en particulier les dérivés de substitution sur les noyaux aromatiques par des radicaux alkyles, oxa- ou aza-alkyles.

18. Procédé selon la revendication 6, **caractérisé en ce que** le mélange réactionnel contient en outre au moins un monomère ou prépolymère capable de polymériser par voie radicalaire et un composé capable de libérer un amorceur de polymérisation radicalaire sous l'effet du rayonnement actinique ou du rayonnement β ou sous l'action de la chaleur.

19. Procédé de modification des propriétés de solubilité d'un polymère possédant des groupements sensibles aux acides, **caractérisé en ce qu'**il consiste à soumettre ledit polymère à un rayonnement actinique ou un rayonnement β, en présence d'un composé selon la revendication 1.

20. Procédé selon la revendication 19, **caractérisé en ce que** le polymère contient des motifs ester ou des motifs aryléther d'alcool tertiaire.

21. Procédé selon la revendication 20, **caractérisé en ce que** le polymère est choisi dans le groupe constitué par les polyacrylates de tertiobutyle, les polyitaconates de tertiobutyle, le poly(tertiobutoxycarbonyloxystyrène), le poly(tertiobutyxostyrène).

22. Procédé selon la revendication 19, **caractérisé en ce qu'**il est mis en oeuvre pour l'amplification chimique de photorésists.

23. Procédé selon la revendication 19, **caractérisé en ce qu'**il est mis en oeuvre à l'aide d'un composé répondant à l'une des formules (III), (VI), (IX), (XV) ou (XVIII).

## Patentansprüche

1. lonische Polymer- oder Oligomerverbindung, bestehend aus einem polykationischen Teil (A⁺)ₚ, der mehrere Oniumeinheiten und eine ausreichende Menge an Anionen X⁻ umfasst, um die elektrische Neutralität der Verbindung sicherzustellen, **dadurch gekennzeichnet, dass**:
- die Oniumeinheiten aus der aus Biaryliodonium-, Arylsulfonium-, Arylacylsulfonium-., Diazonium- und Organometallkationen, die ein Übergangsmetall umfassen, das mit zumindest einem ungesättigten Ring komplexiert ist, der 4 bis 12 Kohlenstoffatome umfasst, bestehenden Gruppe ausgewählt sind;
- X- ein Imidanion [R_{F}SO₂NSO₂R'_{F}]⁻ oder ein Methylanion [R_{F}SO₂C(Q)SO₂R'_{F}]⁻ ist, worin:
1) Q für:
- H-, Cl-, F-, Br- oder CN-;
- einen Alkylrest mit 1 bis 30 Kohlenstoffatomen;
- einen Aryl-, Alkylaryl- oder Arylalkylrest mit 6 bis 30 Kohlenstoffatomen;
- eine R"_{F}-Gruppe oder eine R"_{F}SO₂-Gruppe steht;
2) R_{F} und R'_{F}, sowie R"_{F} sofern X⁻ ein Methylanion ist, unabhängig voneinander aus der aus Fluor, Perhalogenalkyl-Gruppierungen mit 1 bis 30 Kohlenstoffatomen, (Perhalogenalkyl)alkyloxy-Gruppierungen, perhalogenierten cycloaliphatischen Gruppierungen mit 3 bis 30 Kohlenstoffatomen, die gegebenenfalls Heteroatome, ausgewählt aus O und N, enthalten und/oder gegebenenfalls zumindest ein Perhalogenalkyl-Kettenglied tragen, und perhalogenierten Arylgruppierungen mit 6 bis 30 Kohlenstoffatomen bestehenden Gruppe ausgewählt sind; oder
3) R_{F} und R'_{F} zusammen einen zweiwertigen Rest bilden, der zusammen mit der Gruppe -SO₂-N-SO₂- bzw. mit der Gruppe -SO₂-C(Q)-SO₂- einen Ring bildet, oder, sofern X⁻ ein Methylanion ist, R"_{F} mit einem der Reste R_{F} oder R'_{F} einen zweiwertigen Rest bildet, der zusammen mit der Gruppe -SO₂-C-SO₂- bzw. mit der Gruppe -SO₂-C- einen Ring bildet, worin der zweiwertige Rest aus perfluorierten Alkylenresten mit 2 bis 12 Kohlenstoffatomen ausgewählt ist, wobei der dritte vorhandene Rest gegebenenfalls aus den oben unter 2) genannten einwertigen Resten ausgewählt ist;
4) p für die Anzahl der Oniumeinheiten steht.

2. lonisches Polymer nach Anspruch 1, **dadurch gekennzeichnet, dass** das Anion ein Sulfonimid [R_{F}SO₂NSO₂R'_{F}]⁻ oder ein Sulfonylmethyl [R_{F}SO₂C(Q)SO₂R'_{F}]⁻ ist, worin:
- Q aus der aus Alkyl-, Aryl-, Alkylaryl- oder Arylalkylgruppierungen mit höchstens 30 Kohlenstoffatomen, Perfluoralkylsulfonylen mit 1 bis 8 Kohlenstoffatomen und Perfluoralkylresten mit 1 bis 12 Kohlenstoffatomen bestehenden Gruppe ausgewählt ist;
- R_{F} und R'_{F} unabhängig voneinander aus der aus Perfluoralkyl-Gruppierungen mit 1 bis 10 Kohlenstoffatomen ausgewählt sind; oder
- R_{F} und R'_{F} zusammen einen zweiwertigen unverzweigten Perfluoralkylenrest mit 1 bis 8 Kohlenstoffatomen bilden.

3. lonisches Polymer nach Anspruch 1, bestehend aus einem Polyiodoniumsalz, für das eine der folgenden Formeln (I) bis (IV) gilt, oder aus einem Polysulfoniumsalz, für das eine der folgenden Formeln (V) bis (IX) gilt, oder aus einem Polyacylsulfoniumsalz, für das eine der folgenden Formeln (X) bis (XIV) gilt, oder aus einem Polydiazoniumsalz, für das die folgende Formel (XV) gilt, oder aus einem Organometall-Polyoniumsalz, für das eine der folgenden Formeln (XVI) bis (XX) gilt: 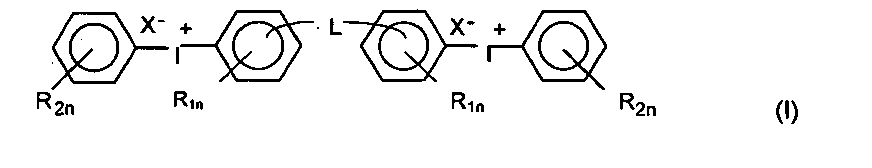 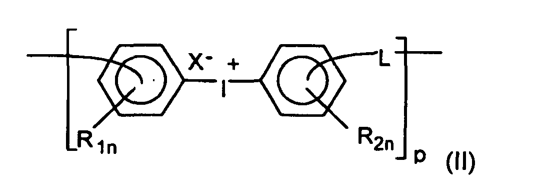 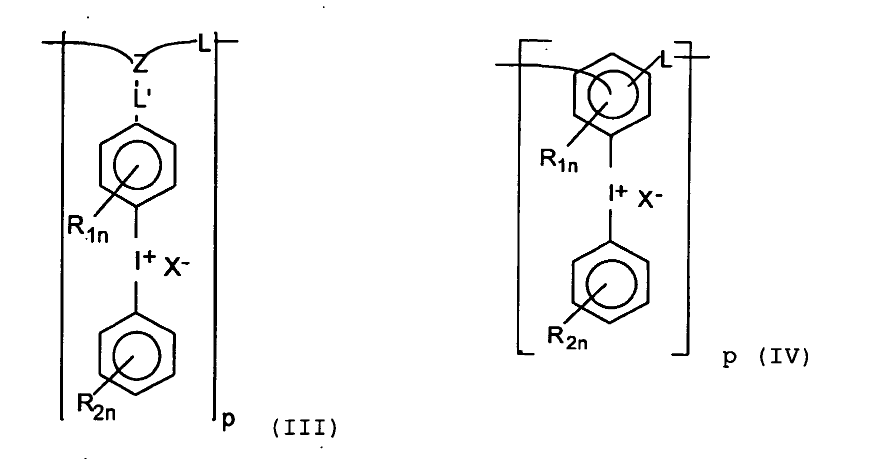 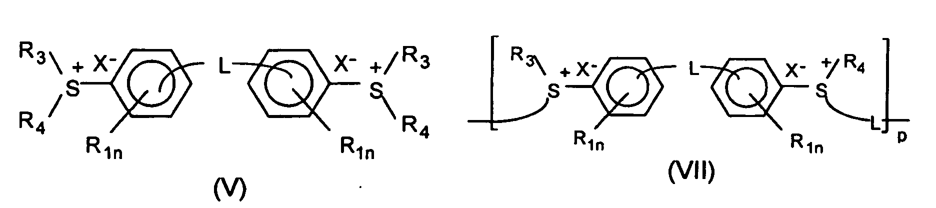 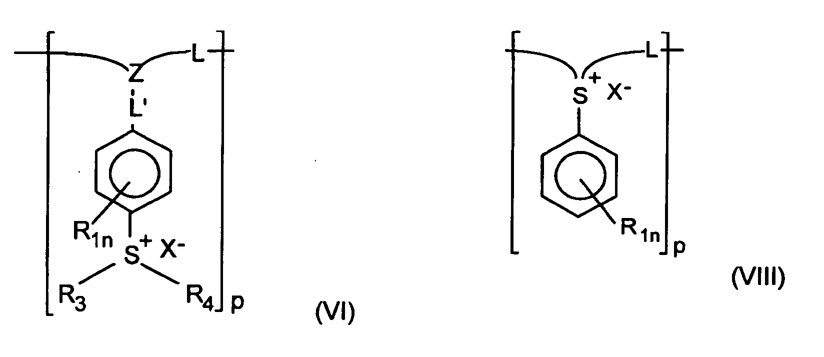 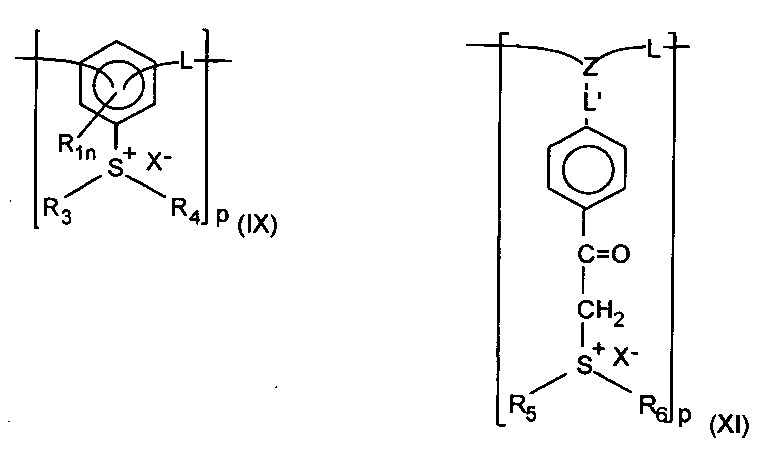 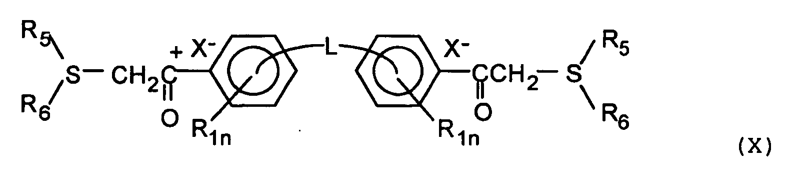 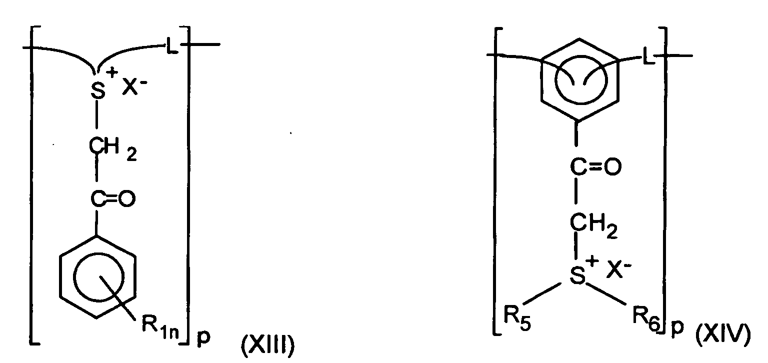 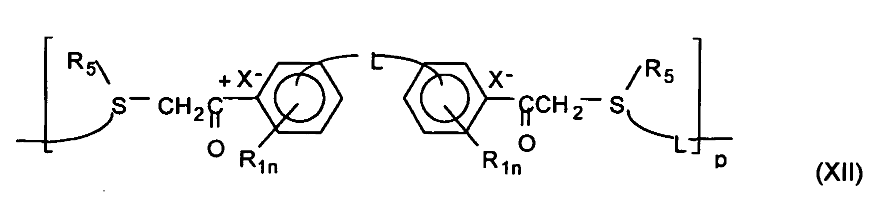 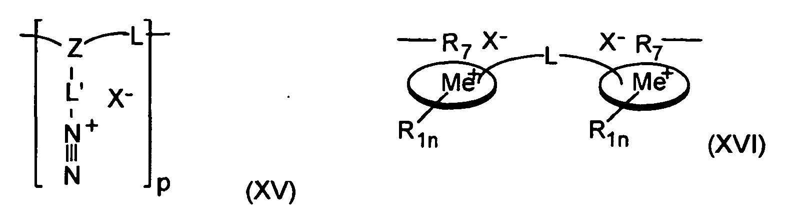 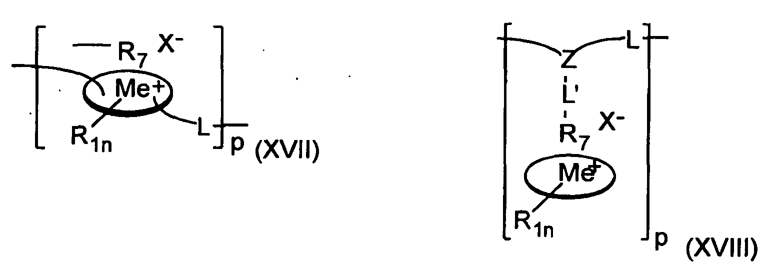 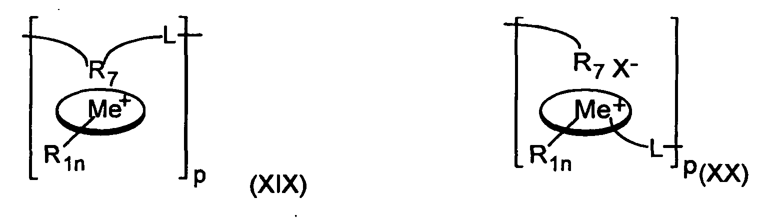 worin:
a1) R₁ₙ für 1 bis 4, vorzugsweise für 1 bis 2, gleiche oder unterschiedliche Gruppierungen steht, die an ein beliebiges der freien Kohlenstoffatome der Arylgruppe gebunden sind, R₂ₙ für 1 bis 4, vorzugsweise für 1 bis 2, gleiche oder unterschiedliche Gruppierungen steht, die an ein beliebiges der freien Kohlenstoffatome der Arylgruppe gebunden sind, und die Gruppierungen R₁ₙ und R₂ₙ sowie R₃ bis R₈ unabhängig voneinander ausgewählt sind aus:
- unverzweigten oder verzweigten Alkyl- oder Arylalkylresten mit 1 bis 30 Kohlenstoffatomen;
- Alkenylresten mit 1 bis 30 Kohlenstoffatomen;
- Aryl- oder Alkylarylresten mit 6 bis 30 Kohlenstoffatomen, einschließlich solchen mit kondensierten Ringen;
- Resten mit 1 bis 30 Kohlenstoffatomen, ausgewählt aus der aus Oxaalkylen, Azaalkylen, Thiaalkylen, Phosphaalkylen, Oxaalkylenen, Azaalkylenen, Thiaalkylenen und Phosphaalkylenen bestehenden Gruppe;
- Resten mit 1 bis 30 Kohlenstoffatomen, die eine Sulfoxidgruppe, eine Sulfongruppe, eine Phosphinoxidgruppe, eine Phosphonatgruppe umfassen, wobei alle diese Reste durch Addition von Sauerstoff an die Schwefelatome oder Phosphoratome erhalten werden;
- aromatischen heterozyklischen oder alizyklischen Resten, die zumindest ein aus der aus O, N, S und P bestehenden Gruppe ausgewähltes Heteroatom umfassen;
- -NO, -CN, -OH, -Cl, -Br, -I, -F;
oder zwei Substituenten, ausgewählt aus den R₁ₙ und den R₂ₙ, und/oder den Substituenten R₃ und R₄ und/oder den Substituenten R₅ und R₆ zusammen einen zweiwertigen Rest bilden, der zusammen mit der Gruppierung, die sie trägt, einen Ring bildet, worin der zweiwertige Rest aus der aus unverzweigten Alkylenresten mit 1 bis 18 Kohlenstoffatomen, Benzo-Biradikalen, die gegebenenfalls zumindest einen Substituenten, vorzugsweise ausgewählt aus der aus Alkylresten, Oxaalkylresten oder Alkenylresten mit 1 bis 10 Kohlenstoffatomen bestehenden Gruppe, aufweisen, und Oxaalkylengruppierungen der Formel -R'-(OCH₂CH₂)q-O-R'- oder - R'-[OCH(CH₃)CH₂]_{q}-O-R'-, worin R' ein unverzweigter Alkylenrest mit 0 bis 18 Kohlenstoffatomen ist und gilt: 1≤q≤22, bestehenden Gruppe ausgewählt ist;
a2) L' für einen zweiwertigen Rest, ausgewählt aus der aus unverzweigten Alkylenresten mit 1 bis 18 Kohlenstoffatomen, substituierten oder unsubstituierten Phenylengruppierungen, Oxaalkylengruppierungen der Formel -R'-(OCH₂CH₂)_{q}-O-R'- oder -R'-[OCH(CH₃)CH₂]_{q}-O-R'-, worin R' ein unverzweigter Alkylenrest mit 0 bis 18 Kohlenstoffatomen ist und gilt: 1≤q≤22, -O-, -S-, >C=O, Siloxangruppierungen -R'-O-[Si(R)₂O]ᵣ-R'- oder -O-[Si(R)₂O]ᵣ- mit 1≤r≤40, worin R' die zuvor genannte Bedeutung hat und R aus der aus unverzweigten Alkylresten mit 1 bis 18 Kohlenstoffatomen, 2-Ethylhexyl, Phenyl (vorzugsweise ist R = CH₃ oder Phenyl) bestehenden Gruppe ausgewählt ist, bestehenden Gruppe, oder für eine direkte Bindung zwischen zwei Kohlenstoffatomen von zwei nicht kondensierten Arylgruppen steht;
a3) L für einen zweiwertigen Rest, ausgewählt aus der unter Punkt a2) definierten Gruppe, steht; oder L für ein Segment steht, das aus zumindest einer nichtionischen Monomereinheit besteht oder eine ionische Gruppierung aufweist, die gegenüber Einwirkung von aktinischer Strahlung nicht empfindlich ist (wobei L in diesem Fall für den mittleren Abstand zwischen den aktiven ionischen Gruppierungen steht);
a4) p für die Anzahl der Grundeinheiten steht und gilt: 2≤p≤1.000;
a5) Z für CH, CR, N, SiR, SiRO₃ steht, worin R aus unverzweigten Alkylresten mit 1 bis 18 Kohlenstoffatomen, 2-Ethylhexyl und Phenyl ausgewählt ist;
a6) Me für ein Übergangsmetall, ausgewählt aus der Gruppe der Übergangselemente der Spalten 3 bis 12 (Zeilen 3 bis 6) des Periodensystems, steht.

4. Verfahren zur Herstellung eines ionischen Polymers nach Anspruch 1, **dadurch gekennzeichnet, dass** es darin besteht, eine Metathese in Wasser oder in einem Gemisch aus Wasser und niederem Alkohol zwischen einem Salz (A⁺X₁⁻) des Polykations (A⁺)ₚ und einer Verbindung A₁⁺X-, die beide im Reaktionsmedium löslich sind, durchzuführen, wobei das Anion X₁ hydrophil ist und das Kation A₁ + aus Alkalimetallen und Erdalkalimetallen ausgewählt ist.

5. Verfahren nach Anspruch 4, **dadurch gekennzeichnet, dass** das Anion X₁-ein Hydroxid, ein Chlorid, ein Bromid, ein Hydrogensulfat, ein Dihydrogenphosphat oder ein Methylsulfonat ist.

6. Verfahren zur Polymerisation oder Vernetzung von Monomeren oder Präpolymeren, die in der Lage sind, kationisch zu reagieren, **dadurch gekennzeichnet, dass** eine Verbindung nach Anspruch 1 als Photoinitiator-Säurequelle verwendet wird, die die Reaktion katalysiert.

7. Verfahren nach Anspruch 6, **dadurch gekennzeichnet, dass** die Monomere aus der aus Verbindungen, die eine zyklische Etherfunktionalität, eine zyklische Thioetherfunktionalität oder eine zyklische Aminfunktionalität tragen, Vinylverbindungen, Vinylethern, Oxazolinen, Lactonen und Lactamen bestehenden Gruppe ausgewählt sind.

8. Verfahren nach Anspruch 6, **dadurch gekennzeichnet, dass** das Präpolymer aus der aus Verbindungen, in denen Epoxygruppierungen von einer aliphatischen Kette, einer aromatischen Kette oder einer heterozyklischen Kette getragen werden, bestehenden Gruppe ausgewählt ist.

9. Verfahren nach Anspruch 6, **dadurch gekennzeichnet, dass** es darin besteht, den Photoinitiator mit zumindest einem Monomer oder Präpolymer zu vermischen, das in der Lage ist, kationisch zu polymerisieren, und das erhaltene Gemisch aktinischer Strahlung oder β-Strahlung auszusetzen.

10. Verfahren nach Anspruch 9, **dadurch gekennzeichnet, dass** das Reaktionsgemisch Strahlung ausgesetzt wird, nachdem es zu einer dünnen Schicht geformt wurde.

11. Verfahren nach Anspruch 6, **dadurch gekennzeichnet, dass** die Menge des verwendeten Photoinitiators zwischen 0,01 und 15 Gew.% des Monomer- oder Präpolymergewichts beträgt.

12. Verfahren nach Anspruch 6, **dadurch gekennzeichnet, dass** der Photoinitiator in Form einer Lösung in einem Lösungsmittel eingesetzt wird, das gegenüber der Polymerisationsreaktion inert ist.

13. Verfahren nach Anspruch 12, **dadurch gekennzeichnet, dass** das inerte Lösungsmittel aus der aus Aceton, Methylethylketon, Acetonitril, Propylencarbonat, γ-Butyrolacton, Etherestern von Mono-, Di-, Triethylen- und -propylenglykolen, Etheralkoholen von Mono-, Di-, Triethylen- oder -propylenglykolen, Phthalsäure-und Zitronensäurestern bestehenden Gruppe ausgewählt ist.

14. Verfahren nach Anspruch 6, **dadurch gekennzeichnet, dass** die Reaktion in Gegenwart eines Lösungsmittels oder eines Verdünnungsmittels durchgeführt wird, das aus einer gegenüber der Polymerisation reaktiven Verbindung besteht.

15. Verfahren nach Anspruch 14, **dadurch gekennzeichnet, dass** die reaktive Verbindung aus der aus Mono- und Divinylethern von Mono-, Di-, Tri-, Tetraethylen- und -propylenglykolen, Trimethylolpropantrivinylether und Dimethanolcyclohexandivinylether, N-Vinylpyrrolidon und Propylencarbonat-2-propenylether bestehenden Gruppe ausgewählt ist.

16. Verfahren nach Anspruch 6, **dadurch gekennzeichnet, dass** dem Reaktionsmedium ein Photosensibilisator zugesetzt wird.

17. Verfahren nach Anspruch 16, **dadurch gekennzeichnet, dass** der Photosensibilisator aus der aus Anthracen, Diphenyl-9,10-anthracen, Perylen, Phenothiazin, Tetracen, Xanthon, Thioxanthon, Isopropylthioxanton, Acetophenon, Benzophenon, 1,3,5-Triaryl-2-pyrazolinen und Derivaten davon, insbesondere Derivaten mit Substitutionen an den aromatischen Ringen in Form von Alkyl-, Oxaalkyl- oder Azaalkylresten, bestehenden Gruppe ausgewählt ist.

18. Verfahren nach Anspruch 6, **dadurch gekennzeichnet, dass** das Reaktionsgemisch weiters zumindest ein Monomer oder Präpolymer, das in der Lage ist, radikalisch polymerisieren, und eine Verbindung enthält, die in der Lage ist, unter Einwirkung von aktinischer Strahlung oder β-Strahlung oder unter Wärmeeinwirkung einen radikalischen Polymerisationsinitiator freizusetzen.

19. Verfahren zur Modifizierung der Löslichkeitseigenschaften eines Polymers, das säureempfindliche Gruppierungen aufweist, **dadurch gekennzeichnet, dass** es darin besteht, das Polymer in Gegenwart einer Verbindung nach Anspruch 1 aktinischer Strahlung oder β-Strahlung auszusetzen.

20. Verfahren nach Anspruch 19, **dadurch gekennzeichnet, dass** das Polymer Estermotive oder Arylethermotive von tertiärem Alkohol enthält.

21. Verfahren nach Anspruch 20, **dadurch gekennzeichnet, dass** das Polymer aus der aus tert-Butylpolyacrylaten, tert-Butylpolyitaconaten, Poly(tert-butoxy-carbonyloxystyrol) und Poly(tert-butoxystyrol) bestehenden Gruppe ausgewählt ist.

22. Verfahren nach Anspruch 19, **dadurch gekennzeichnet, dass** es zur chemischen Amplifikation von Photoresists durchgeführt wird.

23. Verfahren nach Anspruch 19, **dadurch gekennzeichnet, dass** es mithilfe einer Verbindung durchgeführt wird, die einer der Formeln (III), (VI), (IX), (XV) oder (XVIII) entspricht.

## Claims

1. Polymer or oligomer ionic compound made of a polycationic part (A⁺)ₚ comprising a plurality of onium units and a sufficient number of anions X⁻ to provide electrical neutrality to the compound, **characterized in that** :
- the onium units are selected from the group consisting of biaryliodonium, arylsulfonium, arylacylsulfonium, diazonium, organometallic cations comprising a transition metal which is complexed by at least one unsaturated cycle comprising 4 to 12 carbon atoms;
- X⁻ is an imide anion [R_{F}SO₂NSO₂R'_{F}]⁻ or a methylide anion [R_{F}SO₂C (Q) SO₂R'_{F}]⁻ in which :
1) Q represents :
- H-, C1-, F-, Br- or CN-;
- an alkyl radical having 1 to 30 carbon atoms;
- an aryl, alkylaryl or arylakyl radical having 6 to 30 carbon atoms;
- a group R"_{F} or a group R"_{F}SO₂;
2) R_{F} and R'_{F}, as well as R"_{F} when X⁻ is a methylide anion, are independently selected from the group consisting of fluorine, perhaloalkyl groups having 1 to 30 carbon atoms, (perhaloalkyl)alkyloxy groups, perhalogenated cycloaliphatic groups having 3 to 30 carbon atoms possibly containing heteroatoms selected from O and N, and/or possibly comprising at least one perhaloalkyl chain, the perhalogenated aryl having 6 to 30 carbon atoms; or
3) R_{F} and R'_{F} together form a bivalent radical constituting a cycle respectively with group -SO₂-N-SO₂- or with group -SO₂-C(Q)-SO₂-, or when X⁻ is a methylide anion, R"_{F} constitutes with one of the radicals R_{F} or R'_{F} a bivalent radical constituting a cycle respectively with group -SO₂-C-SO₂- or with group -SO₂-C-, said bivalent radical being selected from perfluorinated alkylene radicals having 2 to 12 carbon atoms, the third radical which is possibly present being selected from the monovalent radicals mentioned above in 2);
4) p represents the number of onium units.

2. Ionic polymer according to claim 1, **characterised in that** the anion is a sulfonimide [R_{F}SO₂NSO₂R'_{F}]⁻ or a sulfonylmethylide [R_{F}SO₂C(Q)SO₂R'_{F}]⁻ in which:
- Q is selected from the group consisting of alkyl, aryl, alkylaryl or arylalkyl groups having at most 30 carbon atoms, the perfluoroalkylsulfonyl groups having 1 to 8 carbon atoms and the perfluoroalkyl radicals having 1 to 12 carbon atoms;
- R_{F} et R'_{F} are independently selected from the group consisting of perfluoroalkyl groups having 1 to 10 carbon atoms, or;
- R_{F} et R'_{F} together form a linear perfluoroalkylene radical having 1 to 8 carbon atoms.

3. Ionic polymer according to claim 1 comprising a salt of polyiodonium represented by one of the following Formulae (I) to (IV), or a polysulfonium salt represented by one of the following Formulae (V) to (IX), or a polyacylsulfonium salt represented by one of the following Formulae (X) to (XIV), or a polydiazonium salt represented by the following Formulae (XV), or an organometallic polyonium salt represented by one of the following Formulae (XVI) to (XX): 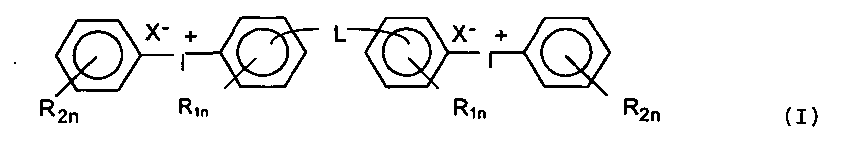 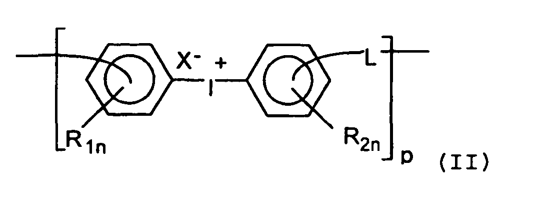 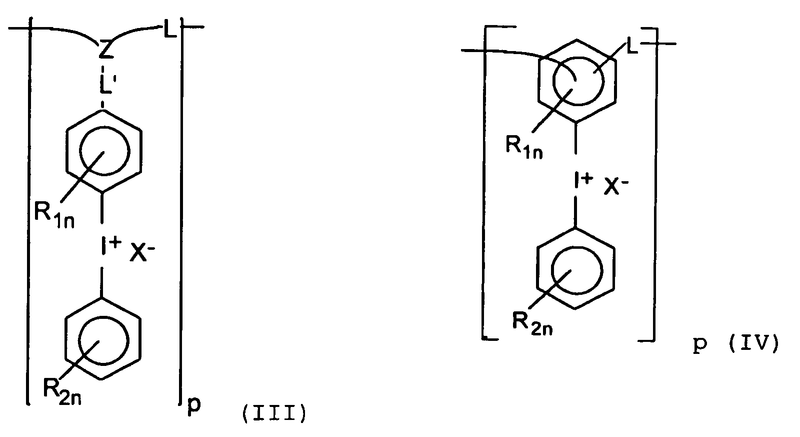 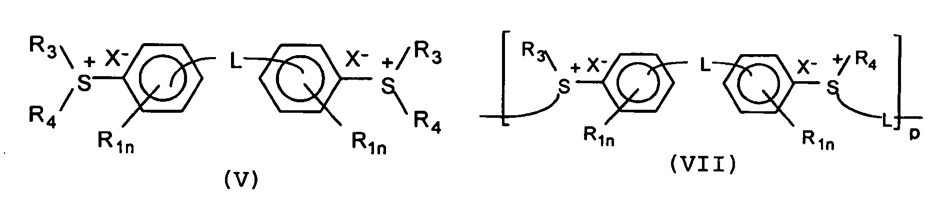 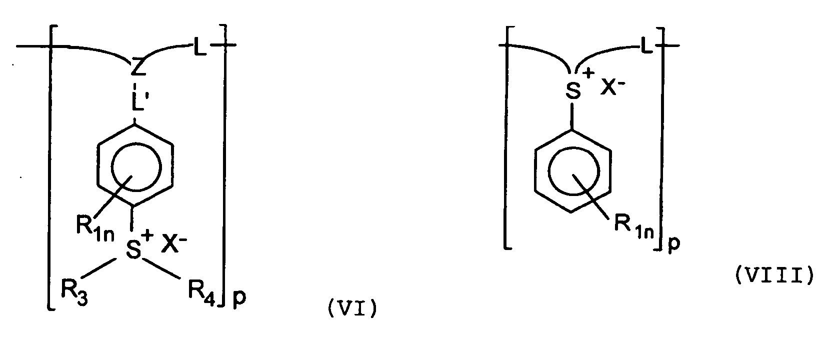 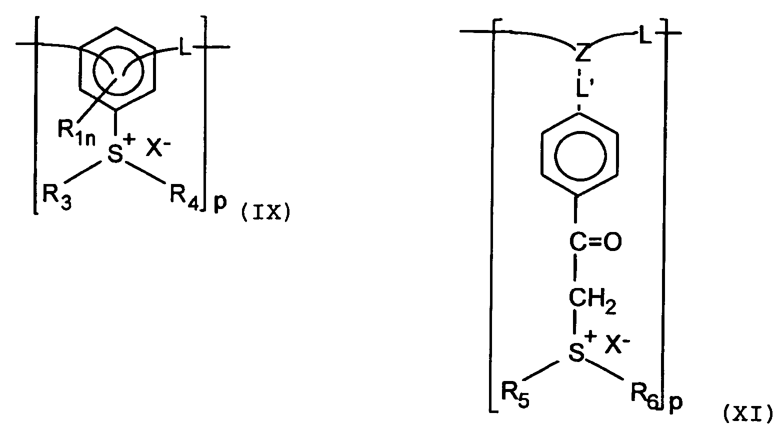 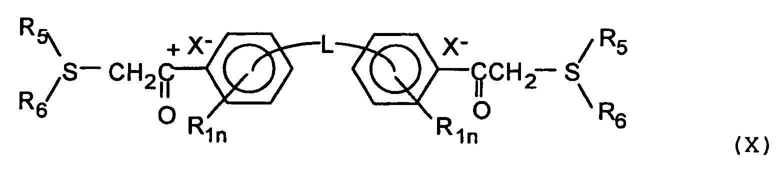 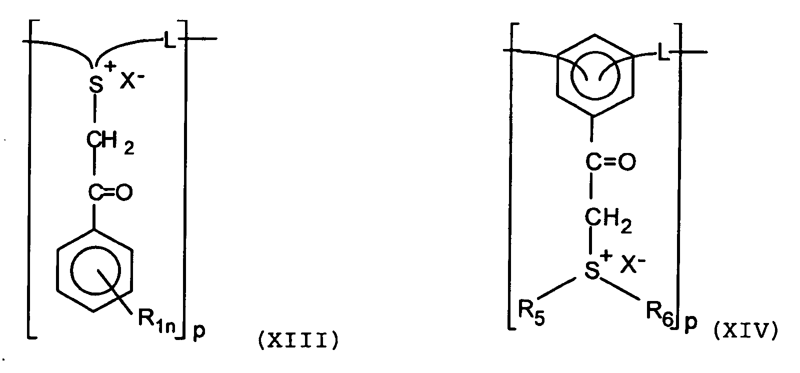 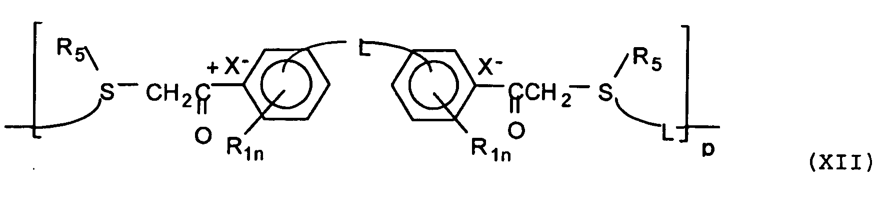 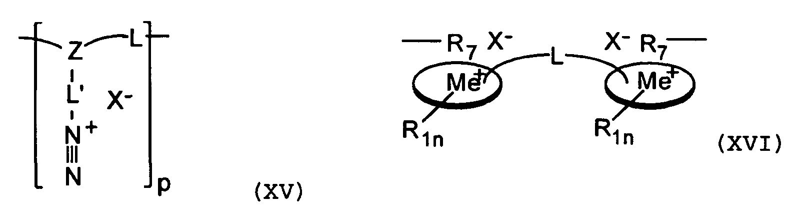 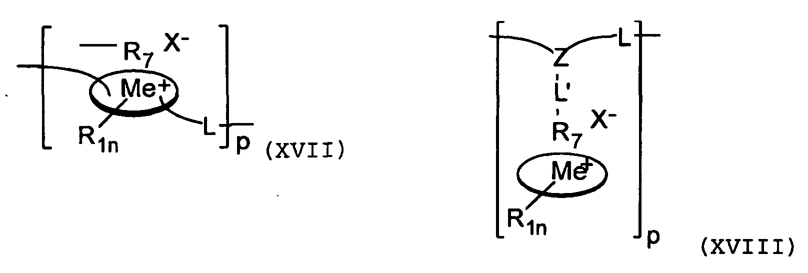 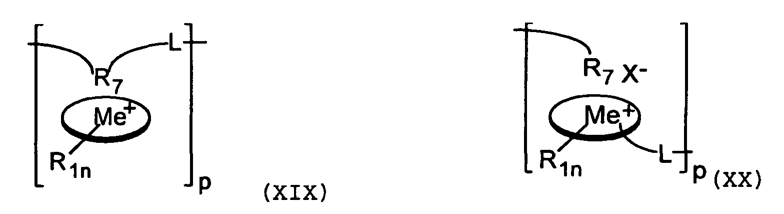 in which:
a1) R₁ₙ represents 1 to 4, preferably 1 to 2 groups, which are identical or different and are bound to any free carbon atom of the aryl group, R₂ₙ represents 1 to 4, preferably 1 to 2 groups which are identical or different and are bound to any free carbon atom of the aryl group, the R₁ₙ and R₂ₙ groups as well as R₃ to R₈ groups being independently selected from:
- linear or branched alkyl or arylalkyl radicals having 1 to 30 carbon atoms;
- alkenyl radicals having 1 to 30 carbon atoms;
- aryl or alkylaryl radicals and aryl or alkylaryl radicals having condensed nuclei having 6 to 30 carbon atoms;
- radicals having 1 to 30 carbon atoms selected from the group consisting of oxaalkyls, azaalkyls, thiaalkyls, phosphaalkyls, oxaalkylenes, azaalkylenes, thiaalkylenes, phosphaalkylenes;
- radicals having 1 to 30 carbon atoms and having a sulfoxide group, a sulfone group, a phosphine oxide group, a phosphonate group, all these radicals being obtained by the addition of oxygen on the sulfur or phosphorus atoms ;
- aromatic or alicyclic heterocyclic radicals comprising at least one heteroatom selected from the group consisting of 0, N, S and P ;
- -NO, -CN, -OH, -Cl, -Br, -I, -F ;
or two substituents selected from R₁ₙ and R₂ₙ and/or substituents R₃ and R₄ and/or substituents R₅ and R₆ together form a bivalent radical which forms a cycle with the group carrying it, said bivalent radical being selected from the group consisting of linear alkylene radicals having 1 to 18 carbon atoms, benzo biradicals possibly carrying at least one substituent, preferably selected from the group consisting of alkyl, oxaalkyl or alkenyl radicals having 1 to 10 carbon atoms, oxaalkylene groups having the formula -R'-(OCH₂CH₂)_{q}-O-R'- or -R'-[OCH(CH₃)CH₂]_{q}-O-R'- in which R' is a linear alkylene radical having 0 to 18 carbon atoms and 1≤q≤22;
a2) L' represents a bivalent radical selected from the group consisting of linear alkylene radicals having 1 to 18 carbon atoms, substituted or non-substituted phenylene groups, oxaalkylene groups having the formula -R'-(OCH₂CH₂)_{q}-O-R'- or -R'-[OCH(CH₃)CH₂]_{q}-O-R'- in which R' is a linear alkylene radical having 0 to 18 carbon atoms and 1≤q≤22, -O-, -S-, >C=O, siloxane groups -R'-O-[Si(R)₂O]ᵣ-R'- or -O-[Si(R)₂O]ᵣ- 1≤r≤90 in which R' has the meaning given above and R is selected from the group consisting of linear alkyl radicals having 1 to 18 carbon atoms, 2-ethylhexyl, phenyl, (preferably R = CH₃ or phenyl) or a direct bond between two carbon atoms of two noncondensed aryl groups;
a3) L represents a bivalent radical selected from the group defined in point a2) above for L' ; or L represents a segment made of at least one non-ionic monomer unit or possessing an ionic group which is not sensitive towards the action of actinic radiation (L in this case representing the average space between the active ionic groups);
a4) p represents the number of recurring units, 2≤p≤1000;
a5) Z represents CH, CR, N, SiR, SiRO₃, R being selected from linear alkyl radicals having 1 to 18 carbon atoms, 2-ethylhexyl and phenyl;
a6) Me represents a transition metal selected from the group of transition elements of column 3 to 12 (lines 3-6) of the Periodic Classification.

4. Process for the preparation of an ionic polymer according to claim 1, **characterized in that** it consists in carrying out metathesis in water or a water/light alcohol mixture, between a salt (A⁺X₁⁻)ₚ of the polycation (A⁺) ₚ and a compound A₁⁺X⁻, both being soluble in the reaction mixture, the anion X₁ having a hydrophilic character, and the cation A₁⁺ being selected from alkali and alkali-earth metals.

5. Process according to claim 4, **characterized in that** the anion X₁⁻ is a hydroxide, a chloride, a bromide, a hydrogenosulfate, a dihydrogenophosphate or a methylsulfonate.

6. Process for the polymerization or cross-linking of monomers or prepolymers capable of undergoing cationic reaction, **characterized in that** a compound according to claim 1 is used as a photoinitiator constituting a source of acid which catalyzes the reaction.

7. Process according to claim 6, **characterized in that** the monomers are selected from the group consisting of compounds which have a cyclic ether function, a cyclic thioether function or a cyclic amine function, vinyl compounds, vinyl ethers, oxazolines, lactones and lactames.

8. Process according to claim 6, **characterized in that** the prepolymer is selected from the group consisting of compounds in which epoxy groups are carried by an aliphatic, aromatic or heterocyclic chain.

9. Process according to claim 6, **characterized in that** it consists in mixing the photoinitiator with at least one monomer or prepolymer which is capable of cationic polymerization, and subjecting the mixture obtained to actinic or β-radiation.

10. Process according to claim 9, **characterized in that** the reaction mixture is treated with radiation after having been converted into a thin layer.

11. Process according to claim 6, **characterized in that** the quantity of photoinitiator used amounts to between 0.01 and 15 % by weight with respect to the weight of the monomer or prepolymer.

12. Process according to claim 6, **characterized in that** the photoinitiator is used in the form of a solution in a solvent which is inert towards the polymerization reaction.

13. Process according to claim 12, **characterized in that** the inert solvent is selected from the group consisting of acetone, methyl-ethyl ketone, acetonitrile, propylene carbonate, γ-butyrolactone, ether-esters of mono-, di-, triethylene or propylene glycols, ether-alcohols of mono-, di-, triethylene or propylene glycols, esters of phthalic acid or citric acid.

14. Process according to claim 6, **characterized in that** the reaction is carried out in the presence of a solvent or a diluent consisting of a compound which is reactive towards polymerization.

15. Process according to claim 14, **characterized in that** the reactive compound is selected from the group consisting of vinyl mono- and diethers of mono-, di-, tri-, tetraethylene or propylene glycols, trivinyl ether trimethylolpropane and divinylether of dimethanol-cyclohexane, N-vinylpyrolidone, 2-propenylether of propylene carbonate.

16. Process according to claim 6, **characterized in that** a photosensitizer is added to the reaction mixture.

17. Process according to claim 16, **characterized in that** the photosensitizer is selected from the group consisting of anthracene, diphenyl-9,10-anthracene, perylene, phenothiazine, tetracene, xanthone, thioxanthone, isopropylthioxantone, acetophenone, benzophenone, 1,3,5-triaryl-2-pyrazolines and derivatives thereof, in particular derivatives which are substituted on the aromatic nuclei by means of alkyl, oxa- or azaalkyl radicals.

18. Process according to claim 6, **characterized in that** the reaction mixture also contains at least one monomer or prepolymer which is capable of free radical polymerisation and a compound capable of releasing a free radical polymerisation initiator under actinic or β-radiation or under heat.

19. Process for the modification of the solubility properties of a polymer having groups sensitive towards acids, **characterized in that** it consists in subjecting said polymer to an actinic or β-radiation, in the presence of a compound according to claim 1.

20. Process according to claim 19, **characterized in that** the polymer contains units of ester or arylether of tertiary alcohol.

21. Process according to claim 20, **characterized in that** the polymer is selected from the group consisting of tertiobutyl polyacrylates, tertiobutyl polyitaconates, poly(tertiobutoxycarbonyloxystyrene), poly(tertiobutoxystyrene).

22. Process according to claim 19, **characterized in that** it is implemented for the chemical amplification of photoresists.

23. Process according to claim 19, **characterized in that** it is implemented with a compound having one of the Formulae (III), (VI), (IX), (XV) or (XVIII).
